# EUROPEAN PATENT APPLICATION

(11) **EP 4 501 895 A1**
(43) Date of publication of application: **05.02.2025**
(21) Application number: 23775099.7
(22) Date of filing: 24.03.2023
(51) Int. Cl.: C07C 29/151, C07C 31/04

(54) **METHOD FOR PRODUCING METHANOL AND APPARATUS FOR PRODUCING METHANOL**

(30) Priority: 25.03.2022 JP 2022050136
(71) Applicant: MITSUBISHI GAS CHEMICAL COMPANY, INC., Chiyoda-ku Tokyo 100-8324 (JP)
(72) Inventor: NAKAGAWA, Ryota, Niigata-shi, Niigata 950-3121 (JP); ABE, Takanori, Tokyo 100-8324 (JP); AKIYOSHI, Shuusuke, Tokyo 100-8324 (JP); KAKIMI, Atsushi, Tokyo 100-8324 (JP)
(74) Representative: Vossius & Partner Patentanwälte Rechtsanwälte mbB
(86) International application number: PCT/JP2023/011879
(87) International publication number: WO 2023/182506

(57) **Abstract**

A method for producing methanol including a synthesis step and a separation step, wherein the method includes: a synthesis loop including at least two of the synthesis steps and at least two of the separation steps, a raw material gas mixing step of obtaining a make-up gas by mixing a hydrogen rich gas obtained from a hydrogen recovery unit to which at least part of unreacted gas separated from a final reaction mixture in a final separation step is supplied with a raw material gas comprising at least hydrogen and carbon dioxide and having M value (M value=[H₂]/([2CO]+[3CO₂])) being 1 or less in the synthesis loop, a distribution ratio controlling step of controlling a distribution ratio of the make-up gas, supplying more than 0 mol % and 100 mol % or less of the make-up gas to a first mixing step in accordance with the distribution ratio, and supplying 0 mol % or more and less than 100 mol % of the make-up gas to a final mixing step, the first mixing step of obtaining a first mixed gas by mixing the make-up gas distributed from the distribution ratio controlling step with a residual gas obtained by removing a part of gas from the final unreacted gas, a first synthesis step of synthesizing the methanol from the first mixed gas, a first separation step of separating the first unreacted gas from the first reaction mixture obtained in the first synthesis step, the final mixing step of obtaining a final mixed gas by finally mixing the unreacted gas with the make-up gas distributed from the distribution ratio controlling step, the final synthesis step of synthesizing the methanol from the final mixed gas, and the final separation step of separating the final unreacted gas from the final reaction mixture obtained in the final synthesis steps.

## Description

### Technical Field

The present invention relates to a method for producing methanol and an apparatus for producing methanol.

### Background Art

Industrial production of methanol is performed by using a fossil fuel as a feedstock, and by allowing a synthesis gas (hereinafter, also referred to as a "synthesis raw material gas" or "raw material gas") mainly comprising carbon monoxide, carbon dioxide and hydrogen, obtained by reforming the fossil fuel, to react on a catalyst. The involved reaction conditions are such that the pressure is 50 to 150 kg/cm², the temperature is 160 to 300°C, and the catalyst used is a catalyst mainly comprising copper/zinc. The methanol synthesis reaction is represented by the following formulas (1) and (2).

CO + 2H₂ → CH₃OH (1)

CO₂ + 3H₂ → CHsOH + H₂O (2)

Recently, a two-stage synthetic methanol synthesis process with multiple reactors has been developed (see, e.g., Patent Documents 1 and 2). In such a two-stage synthesis process, the reaction amount can be controlled by appropriately distributing the raw material gas into the first and second stages to prevent the catalyst from being exposed to high temperature conditions, and the temperature of the catalyst layer can be set to an appropriate temperature range, energy consumption can be reduced, and a high carbon yield can be achieved.

### Citation List

### Patent Documents

[Patent Document 1] WO2016/063872A
[Patent Document 2] WO2017/175760A

### Summary of Invention

### Problem to be Solved

In recent years, international efforts to realize a decarbonisation society have become more active. In response to these efforts, it is necessary to develop a process that takes environmental circulation into account in the synthesis of methanol. For example, waste materials such as waste plastics and eco-recycled products, as well as biomass, provide not only hydrogen but also methanol raw materials such as CO and CO₂. Based on such circumstance, as part of methanol synthesis that takes environmental recycling into consideration, the use of environmental circular gases such as synthesis gas derived from waste wood or biomass as a feedstock gas is considered. Environmental circular gases tend to have low hydrogen concentrations and high CO concentrations, and when each component (molar ratio) of H₂, CO, and CO₂ in a gas is expressed as M value = [H₂]/([2CO]+[3CO₂]), the M value is often lower than 1 (hereinafter, gases with M values lower than 1 are sometimes referred to as "low M value gases").

As noted above, CO concentrations tend to be high in many low M value gases. Gases with high CO concentrations have high reactivity for methanol synthesis reactions and have the potential for efficient methanol synthesis. However, since the methanol synthesis reaction is an exothermic reaction, care must be taken to avoid excessive heat generation in the catalyst layer. Exposure of the catalyst to high temperature conditions is undesirable in terms of catalyst degradation and by-product formation. In this regard, in the above Patent Documents 1 and 2, a two-stage synthesis process is used, and by appropriately distributing the raw material gas between the first and second stages and controlling the reaction amount, it is possible to prevent the catalyst from being exposed to high temperature conditions.

On the other hand, low M value gases lack hydrogen when used for methanol synthesis. However, there are few examples of low M value gases with low hydrogen content being utilized in methanol synthesis processes with largely required hydrogen stoichiometry, and there is a need to develop efficient production methods for methanol production using low M value gases. Specifically, one of the challenges in these methanol synthesis processes is to increase energy efficiency by lowering the synthesis pressure to reduce the load on the compressor.

In order to solve the above-mentioned problems, the purpose of the present invention is to provide a method for producing methanol and an apparatus for producing methanol, each of which can use low M value gas in the synthesis of methanol and has excellent energy efficiency.

### Solution to Problem

The present inventors made a diligent study in order to solve the above-mentioned problems. As a result, they found that a specific process can synthesize methanol while maintaining low energy consumption, even when low M value gas is used.

<1> A method for producing methanol comprising:
   a synthesis step of synthesizing methanol from a synthesis gas comprising hydrogen, carbon monoxide and carbon dioxide; and
   a separation step of separating an unreacted gas from a reaction mixture obtained by passing through the synthesis steps,
   wherein the method comprises:
      a synthesis loop comprising at least two of the synthesis steps and at least two of the separation steps,
      a raw material gas mixing step of obtaining a make-up gas by mixing a hydrogen rich gas obtained from a hydrogen recovery unit to which at least part of unreacted gas separated from a final reaction mixture in a final separation step is supplied with a raw material gas comprising at least hydrogen and carbon dioxide and having M value (M value=[H₂]/([2CO]+[3CO₂])) being 1 or less in the synthesis loop,
      a distribution ratio controlling step of controlling a distribution ratio of the make-up gas, supplying more than 0 mol % and 100 mol % or less of the make-up gas to a first mixing step in accordance with the distribution ratio, and supplying 0 mol % or more and less than 100 mol % of the make-up gas to a final mixing step,
      the first mixing step of obtaining a first mixed gas by mixing the make-up gas distributed from the distribution ratio controlling step with a residual gas obtained by removing a part of gas from the final unreacted gas,
      a first synthesis step of synthesizing the methanol from the first mixed gas,
      a first separation step of separating the first unreacted gas from the first reaction mixture obtained in the first synthesis step,
      the final mixing step of obtaining a final mixed gas by finally mixing the unreacted gas with the make-up gas distributed from the distribution ratio controlling step,
      the final synthesis step of synthesizing the methanol from the final mixed gas, and
      the final separation step of separating the final unreacted gas from the final reaction mixture obtained in the final synthesis steps.
<2> The method for producing methanol according to <1>, wherein the M value is 0.8 or more and 0.95 or less.
<3> The method for producing methanol according to <1> or <2>, wherein the raw material gas comprises an environmental circular gas.
<4> The method for producing methanol according to any one of <1> to <3>, wherein 50 mol % or more and 100 mol % or less of the make-up gas is distributed into the first mixing step.
<5> The method for producing methanol according to any one of <1> to <4>, wherein the make-up gas obtained in the raw material gas mixing step is pressurized.
<6> The method for producing methanol according to any one of <1> to <5>, wherein a circulating ratio that is a ratio of a molar flow rate of the make-up gas to a molar flow rate of the final unreacted gas mixed with the make-up gas is 0.6 to 2.0.
<7> The method for producing methanol according to <6>, wherein the circulation ratio is 0.8 to 1.5.
<8> An apparatus for producing methanol comprising:
   a reactor for synthesizing methanol from a synthesis gas comprising hydrogen, carbon monoxide and carbon dioxide; and
   a separator for separating an unreacted gas from a reaction mixture obtained in the reactors,
   wherein the apparatus comprises:
      a synthesis loop comprising at least two of the reactors and at least two of the separators,
      a raw material gas mixing unit for obtaining a make-up gas by mixing a hydrogen rich gas obtained from a hydrogen recovery unit to which at least part of unreacted gas separated from a final reaction mixture in a final separator is supplied with a raw material gas comprising at least hydrogen and carbon dioxide and having M value (M value=[H₂]/([2CO]+[3CO₂])) being 1 or less in the synthesis loop,
      a distribution ratio controller for controlling a distribution ratio of the make-up gas, supplying more than 0 mol % and 100 mol % or less of the make-up gas to a first mixing unit in accordance with the distribution ratio, and supplying 0 mol % or more and more than 100 mol % of the make-up gas to a final mixing unit,
      the first mixing unit for obtaining a first mixed gas by mixing the make-up gas distributed from the distribution ratio controller with a residual gas obtained by removing a part of gas from the final unreacted gas,
      a first reactor for synthesizing the methanol from the first mixed gas,
      a first separator for separating the first unreacted gas from the first reaction mixture obtained in the first reactor,
      the final mixing unit for obtaining a final mixed gas by finally mixing the unreacted gas with the make-up gas distributed from the distribution ratio controller,
      the final reactor for synthesizing the methanol from the final mixed gas, and
      the final separator for separating the final unreacted gas from the final reaction mixture obtained in the final reactors.

### Advantageous Effects of Invention

According to the present invention, it is possible to provide a method for producing methanol and an apparatus for producing methanol, each of which can use low M value gas in the synthesis of methanol and has excellent energy efficiency.

### Brief Description of Drawings

Figure 1 is a schematic diagram showing an example of an apparatus used for the method for producing methanol in the present invention.
Figure 2 is a schematic diagram showing an example of an apparatus used for the method for producing methanol as a comparative example (single-stage synthesis process).
Figure 3 is a schematic diagram showing another example of an apparatus used for the method for producing methanol as a comparative example (without hydrogen recovery unit).
Figure 4 is a table showing the material balance in each line corresponding to Example 1, Comparative Example 1, and Comparative Example 2.
Figure 5 is a table showing the material balance in each line corresponding to Example 2-1 and Comparative Example 4.
Figure 6 is a table showing the material balance in each line corresponding to Example 3 and Comparative Example 5.
Figure 7 is a graph showing the results of Example 4 for the relationship between the distribution ratio and circulation ratio controlling and energy consumption.
Figure 8 is a graph showing the results of Example 5 for the relationship between the M value of the raw material gas and energy consumption.
Figure 9 is a table showing the material balance in each line corresponding to Example 5-2, Example 5-6, and Example 5-10.
Figure 10 is a table showing the material balance in each line corresponding to Example 5-15 and Example 5-19.

### Description of Embodiments

Hereinafter, the mode for carrying out the present invention (hereinafter, simply referred to as the present embodiment) is described in detail with reference to the accompanying drawings if necessary, but the present invention is not limited to the following present embodiment. The present invention can be modified in various ways within the scope not departing from the gist of the present invention. In the accompanying drawings, the same elements will be denoted by the same symbols, and the duplicated descriptions will be omitted. The positional relations such as up, down, left and right are based on the positional relations shown in the drawings, unless otherwise specified. Moreover, the dimensional proportions in the drawings are not limited to the proportions shown in the drawings.

### <<Method for producing methanol of the present embodiment>>

The method for producing methanol of the present embodiment (hereinafter, referred to as a "methanol production method" or "production method") includes:
a synthesis step of synthesizing methanol from a synthesis gas including hydrogen, carbon monoxide and carbon dioxide; and
a separation step of separating an unreacted gas from a reaction mixture obtained by passing through the synthesis steps,
wherein the method includes:
   a synthesis loop including at least two of the synthesis steps and at least two of the separation steps,
   a raw material gas mixing step of obtaining a make-up gas by mixing a hydrogen rich gas obtained from a hydrogen recovery unit to which at least part of unreacted gas separated from a final reaction mixture in a final separation step is supplied with a raw material gas including at least hydrogen and carbon dioxide and having M value (M value=[H₂]/([2CO]+[3CO₂])) being 1 or less in the synthesis loop,
   a distribution ratio controlling step of controlling a distribution ratio of the make-up gas, supplying more than 0 mol % and 100 mol % or less of the make-up gas to a first mixing step in accordance with the distribution ratio, and supplying 0 mol % or more and less than 100 mol % of the make-up gas to a final mixing step,
   the first mixing step of obtaining a first mixed gas by mixing the make-up gas distributed from the distribution ratio controlling step with a residual gas obtained by removing a part of gas from the final unreacted gas,
   a first synthesis step of synthesizing the methanol from the first mixed gas,
   a first separation step of separating the first unreacted gas from the first reaction mixture obtained in the first synthesis step,
   the final mixing step of obtaining a final mixed gas by finally mixing the unreacted gas with the make-up gas distributed from the distribution ratio controlling step,
   the final synthesis step of synthesizing the methanol from the final mixed gas, and
   the final separation step of separating the final unreacted gas from the final reaction mixture obtained in the final synthesis steps.

The apparatus for producing methanol of the present embodiment includes a reactor for synthesizing methanol from a synthesis gas including hydrogen, carbon monoxide and carbon dioxide; and
a separator for separating an unreacted gas from a reaction mixture obtained in the reactors,
wherein the apparatus includes:
   a synthesis loop including at least two of the reactors and at least two of the separators,
   a raw material gas mixing unit for obtaining a make-up gas by mixing a hydrogen rich gas obtained from a hydrogen recovery unit to which at least part of unreacted gas separated from a final reaction mixture in a final separator is supplied with a raw material gas including at least hydrogen and carbon dioxide and having M value (M value=[H₂]/([2CO]+[3CO₂])) being 1 or less in the synthesis loop,
   a distribution ratio controller for controlling a distribution ratio of the make-up gas, supplying more than 0 mol % and 100 mol % or less of the make-up gas to a first mixing unit in accordance with the distribution ratio, and supplying 0 mol % or more and more than 100 mol % of the make-up gas to a final mixing unit,
   the first mixing unit for obtaining a first mixed gas by mixing the make-up gas distributed from the distribution ratio controller with a residual gas obtained by removing a part of gas from the final unreacted gas,
   a first reactor for synthesizing the methanol from the first mixed gas,
   a first separator for separating the first unreacted gas from the first reaction mixture obtained in the first reactor,
   the final mixing unit for obtaining a final mixed gas by finally mixing the unreacted gas with the make-up gas distributed from the distribution ratio controller,
   the final reactor for synthesizing the methanol from the final mixed gas, and
   the final separator for separating the final unreacted gas from the final reaction mixture obtained in the final reactors.

The production method of the present embodiment is a synthesis method using a multi-stage synthesis process and includes a raw material gas mixing step of mixing a hydrogen rich gas recovered from a part of the final unreacted gas with the raw material gas, and a distribution ratio controlling step of controlling a distribution ratio of the make-up gas mixed with an unreacted gas in the first mixing step and the final mixing step. In the production method of the present embodiment, by mixing the raw material gas with hydrogen rich gas and controlling the distribution ratio of the make-up gas supplied to each mixing step according to various conditions, for example, the M value of the synthesis gas supplied to each synthesis step can be adjusted within a desired range without increasing compressor power and decreasing energy efficiency. As a result, the production method of the present embodiment can synthesize methanol with excellent energy efficiency even when low M\ value gas is used as the raw material gas.

Furthermore, according to the production method of the present embodiment, the M value of the synthesis gas at the reactor inlet can be easily controlled to the desired range while maintaining low energy consumption by controlling the distribution ratio of the synthesis gas supplied to each synthesis step. In this regard, as disclosed in, for example, Hiromichi Arai (1978), "Hydrogenation of Carbon Monoxide with Solid Catalysts (I)," Oil Chemistry Vol. 27, No. 8, pp. 491-500), etc., it is known that by-products (ethanol, acetone, methyl ethyl ketone, paraffin, etc.) produced in methanol synthesis increase or decrease depending on CO content and H/C ratio. Therefore, according to the present invention, the amount of by-product generation can be controlled by adjusting the make-up gas distribution ratio, while suppressing the increase in energy consumption.

As described above, the methanol production method and apparatus for producing methanol of the present embodiment make the methanol synthesis process highly efficient (i.e., suppresses the increase in compressor power) while using highly reactive low-M value gas (= high CO concentration gas), and control the amount of by-product generation while maintaining low energy consumption. In addition, according to the methanol production method and apparatus for producing methanol of the present embodiment, the methanol synthesis process can be made more efficient even when environmental circular gases such as biomass-derived gases are used as raw material gas, for example, thus contributing to the realization of a sound material-cycle society.

The methanol production method and apparatus for producing methanol of the present embodiment are described below.

### <Synthesis step and separation step>

The method for producing methanol of the present embodiment is a method for producing methanol including: a synthesis step of synthesizing methanol from a synthesis gas including hydrogen, carbon monoxide and carbon dioxide; and a separation step of separating an unreacted gas from a reaction mixture obtained by passing through the synthesis step. In these steps, a reactor for synthesizing methanol from synthesis gas containing hydrogen, carbon monoxide, and carbon dioxide, and a separator for separating unreacted gas from the reaction mixture obtained in the reactor can be used.

### (Synthesis step)

In the present embodiment, a "synthesis step" means a step of synthesizing methanol from a synthesis gas including hydrogen, carbon monoxide and carbon dioxide. The production method of the present embodiment includes at least two synthesis steps, the first synthesis step and the final synthesis step. For example, in the case where the production method of the present embodiment includes three or more synthesis steps (n ≥ 3), the production method includes the first synthesis step, ..., the "(n-1)th" synthesis step, and the final synthesis step.

Hereinafter, similarly, when referred to as "first ..." and "final ...," respectively, and when the production method of the present embodiment includes three or more synthesis steps (n≥3), the production method includes "first ...", ..., "(n-1)th ...", and "final ..." steps. Details of each synthesis step are described below.

### (Synthesis gas)

In the present embodiment, "synthesis gas" means a generic term for gases that contain hydrogen, carbon monoxide, and carbon dioxide and are subject to the methanol synthesis reaction. In the present embodiment, the "synthesis gas" includes, for example, the raw material gas, make-up gas, each mixed gas (the first mixed gas, the final mixed gas, etc.), and part of unreacted gas, as described below.

### (Separation Step)

In the present embodiment, "separation step" means a generic term for the step of separating unreacted gas from the reaction mixture obtained through the synthesis step. The production method of the present embodiment includes at least two separation steps (the first separation step and the final separation step). In the present embodiment, for example, at least one of the at least two separation steps that the synthesis loop has can separate, in a gas-liquid separator, the liquid containing methanol produced by cooling the gaseous reaction mixture. Details of each separation step are described below.

### (Reaction mixture)

In the present embodiment, "reaction mixture" means a generic term for an outlet component of the synthesis step, which is a mixture of the components resulting from the reaction in the synthesis step and unreacted components, including at least methanol and unreacted gas. In the present embodiment, the "reaction mixture" includes, for example, the first reaction mixture and final reaction mixture described below.

### (Unreacted gas)

In the present embodiment, "unreacted gas" means a generic term for unreacted gas separated from the reaction mixture in the separation step, and includes at least hydrogen, carbon monoxide, and carbon dioxide. For example, the "unreacted gas" in the present embodiment includes the first unreacted gas and final unreacted gas described below.

### <Synthesis loop>.

In the present embodiment, "synthesis loop" includes at least two synthesis steps and at least two separation steps. The synthesis loop is formed in the form that gas that has passed through at least one synthesis step and at least one separation step further passes through the final synthesis step and the final separation step, and unreacted gas separated in the final separation step is mixed with make-up gas and used as the first mixed gas in the first synthesis step. In the synthesis loop, at least two reactors and at least two separators can be used.

Regarding the "inlet" among the inlet and outlet of material of the synthesis loop, make-up gas, a mixture of low M value gas and hydrogen rich gas, is introduced into the synthesis loop from the mixing step prior to each synthesis step after the distribution ratio is controlled in the distribution ratio controlling step and divided into multiple flows. Regarding the "outlet" among the inlet and outlet of material, the reaction products in the reaction mixture are separated and extracted out of the synthesis loop in the separation step, and a part of the final unreacted gas is taken out of the synthesis loop to prevent the accumulation of inert components.

The unreacted gases from each separation stage are led to the next mixing, synthesis, and separation steps, forming a synthesis loop in which each unreacted gas can be introduced into all reactors in series. The synthesis loop may include a preheating step, a pressurizing step, a depressurizing step, and a cooling step.

In the preheating step, a preheater may be used to raise the temperature of synthesis gas supplied to the synthesis step. The preheater includes, for example, a heat exchanger that exchanges heat with the reaction mixture (usually in gaseous form) obtained through each synthesis step in order to raise the temperature of the synthesis gas supplied to the synthesis step to a predetermined temperature. The heat exchanger such as a preheater can also be used to cool the reaction mixture.

In the pressuring step, it is preferable to have at least one circulator for pressurizing. In the pressurizing step, the temperature required for preheating each mixed gas, for example, can be lowered because the temperature can be increased due to the pressuring. When the temperature used for preheating can be lowered, the temperature difference between the low-temperature fluid and the high-temperature fluid in the preheater will be increased, and the amount of heat exchange (heat recovery) in the preheater can be increased or the size of the heat exchanger can be reduced. It is preferable to introduce a fluid with a high degree of dryness into the circulator. More specifically, it is preferable to increase the dryness by preheating the fluid with saturated condensable gas after the separation step, or by mixing a high dryness fluid (e.g., make-up gas), or both. This allows for more suppression of condensation droplet generation in the circulator, which further prevents mechanical failure and increased energy loss.

In addition, the synthesis loop may include a depressurizing step. In the depressurizing step, an unreacted can be depressurized prior to mixing with the make-up gas. For example, the depressurizing step can be a final unreacted gas depressurizing step in which the final unreacted gas obtained in the final separation step is depressurized before the first mixing step. This allows the pressure in the previous final separation step to be increased, thus further improving the gas-liquid separation efficiency in the final separation step. Similarly, the depressurizing step may be an unreacted gas depressurizing step to depressurize the unreacted gas obtained in each separation step.

### <Raw material gas mixing step>

In the present embodiment, the raw material gas mixing step is a step in which, in the synthesis loop, at least a portion of the final unreacted gas separated from the final reaction mixture in the final separation step is supplied to a hydrogen recovery unit, and the hydrogen rich gas obtained from the hydrogen recovery unit is mixed with raw material gas containing at least hydrogen and carbon dioxide and having an M value (M value = [H₂]/([2CO]+[3CO₂])) of 1 or less to obtain the make-up gas. In this step, by mixing the raw material gas having the M value of 1 or less with hydrogen rich gas, the unreacted gas, synthesis pressure, and compressor load can be reduced compared to the case where the raw material gas with the M value exceeding 1 is used, or the raw material gas with the M value of 1 or less is used without mixing hydrogen rich gas.

### (Final separation step)

In the present embodiment, "final separation step" is a step to separate the final unreacted gas from the final reaction mixture obtained in the final synthesis step, and at least part of the unreacted gas in the outlet gas (gaseous reaction mixture) from the final synthesis step is separated in the final separation step. Details of the final separation step are described below.

### (Final unreacted gas)

In the present embodiment, "final unreacted gas" is unreacted gas separated from the final reaction mixture in the final separation step, and at least part of the final unreacted gas is supplied to the hydrogen recovery unit. At least part of the final unreacted gas includes at least hydrogen, carbon monoxide, and carbon dioxide. A part of the final unreacted gas may be discharged out of the system as a "purge gas" to prevent accumulation of inert components.

### (Hydrogen recovery unit)

In the present embodiment, "hydrogen recovery unit" is a unit to which at least part of the final unreacted gas is supplied and from which hydrogen rich gas with a high hydrogen concentration is discharged. The hydrogen recovery unit may be configured i) such that to which a part of the final unreacted gas described above is supplied, and to separate the hydrogen rich gas to be mixed with the make-up gas from the gas to be discharged outside the system, or ii) such that to which all of the final unreacted gas is supplied and to separate the hydrogen rich gas from the unreacted gas to be mixed with the make-up gas. In the present embodiment, the configuration described in i) above is preferred from the viewpoint of the M value of the make-up gas and the prevention of accumulation of inert components. In the configuration of ii) above, the configuration may be such that a part of the unreacted gas after being separated from the hydrogen rich gas by the hydrogen recovery unit is discharged outside the system.

The hydrogen recovery unit is not limited to any unit capable of selectively recovering hydrogen gas from the final unreacted gas. For example, a pressure swing adsorption (PSA) unit or other known hydrogen recovery means may be selected and used as appropriate. The hydrogen recovery capacity of the hydrogen recovery unit is not particularly limited, but for example, the hydrogen separation ratio is preferably 50 mol% or more, and more preferably 80 mol% or more.

### (Hydrogen rich gas)

In the present embodiment, "hydrogen rich gas" is a gas containing hydrogen recovered by the hydrogen recovery unit. The hydrogen concentration of the hydrogen rich gas should be 80 to 100 vol%, and 90 to 100 vol% is more preferred, in order to effectively increase the M value of the make-up gas.

### (Raw material gas)

In the present embodiment, "raw material gas" means a synthetic gas that contains at least hydrogen and carbon dioxide and has the M value = [H₂]/([2CO]+[3CO₂]) of 1 or less. For example, so-called "fossil fuel-derived gases" such as reformed natural gas, partially oxidized natural gas, and coal gasification gas, as well as "environmental circular gases" such as plastic gasification gas derived from waste materials (e.g., waste plastics and environmental recyclable products), biomass gasification gas derived from biomass, and gas derived from digestion gas (methane fermentation gas) can be used as the raw material gas, as long as the M value is 1 or less. From the viewpoint of promoting environmental recycling, it is preferable to include environmental circular gases as raw material gases. The "waste materials" and "biomass" include, for example, waste plastics, environmental recyclable products, waste biomass, unutilized biomass, and resource crops, such as livestock waste, food waste, waste paper, black liquor (pulp mill waste), sewage sludge, human waste sludge, construction wood, lumber mill residues, rice straw, wheat straw, rice husks, forest residues, sugar resources (sugarcane, etc.), starch resources (corn, etc.), oil and fat resources (rapeseed, etc.), willow, poplar, switchgrass, and so on. Plastic gasification gas derived from waste materials and biomass gasification gas derived from biomass can be obtained from these waste materials and biomass using known means.

However, the raw material gas in the present embodiment is not limited to the environmental circular gases and fossil fuel-derived gases exemplified above, but can be, for example, a combination of two or more gases selected from environmental circular gases, fossil fuel-derived gases, gases derived from CO₂ sources (described below), and gases derived from H₂ sources (described below). Gases obtained by adjusting the composition of those gases by means of shift reactors, steam reforming, adsorption facilities, etc. can be used. For example, fossil fuel-derived gases and environmental circular gases may be produced by combining gases derived from CO₂ and H₂ sources, as described below. Furthermore, a gas that contains hydrogen and carbon dioxide gas but does not contain carbon monoxide may be used as the raw material gas.

The aforementioned gases derived from CO₂ sources are gases containing at least CO₂, which may include, for example, CO₂ from thermal power generation, CO₂ from biomass power generation, CO₂ associated with natural gas, by-product CO₂ from chemical production, CO₂ from digested sewage treatment gas, CO₂ from methane fermentation, CO₂ from mineral processes (cement, aluminum, etc.), CO₂ from the steel industry, CO₂ from waste combustion processes, etc.

The aforementioned gases derived from H₂ sources are gases containing at least H₂, which may include, for example, hydrogen from petroleum refining facilities, hydrogen from chemical processes (e.g., from fossil fuels, by-product hydrogen, surplus hydrogen, etc.), blue hydrogen (hydrogen from Carbon Dioxide Capture and Storage (CCS)), electrolytic hydrogen (alkaline water electrolysis, brine electrolysis, electrolytic hydrogen using renewable energy, other electrolytic hydrogen).

In the methanol synthesis method of the present embodiment, the gas before being mixed with the hydrogen rich gas obtained from the hydrogen recovery unit is the raw material gas, and the hydrogen rich gas is mixed with the raw material gas to form the make-up gas.

The raw material gas is not particularly limited as long as it is the "low M value gas" with the M value of 1 or less. However, from the viewpoint of using an environmental circular gas and improving energy efficiency by reducing the load on the compressor, for example, the M value of the raw material gas is preferably 1.00 or less, more preferably 0.8 or more and 0.95 or less, and especially preferably 0.85 or more and 0.95 or less.

### (Make-up gas)

In the present embodiment, "make-up gas" means a synthesis gas in which the raw material gas and the hydrogen rich gas obtained from the hydrogen recovery unit are mixed. In other words, when the raw material gas is mixed with the hydrogen rich gas obtained from the hydrogen recovery unit, it becomes make-up gas. The M value of the make-up gas before it is divided into multiple flows (before introduction to the synthesis loop) is not particularly limited, but, for example, is preferably more than 1.0 and I 2.0 or less, is more preferably 1.01 or more and 2.00 or less, and further preferably 1.05 or more and 1.50 or less.

### -Pressurizing of make-up gas-

In the raw material gas mixing step, the resulting make-up gas can be pressurized. For example, the make-up gas can be pressurized by a compressor or the like. The make-up gas can be pressurized, for example, before the make-up gas is divided into multiple flows.

The make-up gas is preferably pressurized to the reaction pressure. For example, the reaction pressure of the make-up gas can be 4.9 to 14.7 MPa-G (50 to 150 kg/cm²-G), and 5.9 to 10.8 MPa-G (60 to 110 kg/cm² -G) is preferred.

### <Distribution ratio controlling Step>

In the present embodiment, "distribution ratio controlling step" is a step for controlling the distribution ratio of the make-up gas and supplying more than 0 mol% and less than 100 mol% of the make-up gas to the first mixing step and 0 mol% or more and less than 100 mol% of the make-up gas to the final mixing step, according to the distribution ratio. For this step, a distribution ratio controller having, for example, a valve electrically connected to a control unit can be used. In the present embodiment, the make-up gas is divided into multiple flows before being introduced into the synthesis loop, and is introduced into the synthesis loop as part of the synthesis gas in the multiple synthesis steps present in the synthesis loop. In the present embodiment, the distribution ratio of the make-up gas supplied to the first mixing step and the final mixing step can be controlled. In other words, in the present embodiment, at least part of the make-up gas may be supplied to the first mixing step and at least part of the residual amount of make-up gas may be supplied to the final mixing step, or all (100 mol%) of the make-up gas may be supplied to the first mixing step only. Furthermore, when the production method of the present embodiment includes three or more synthesis steps (n≥3), at least part of the make-up gas may be supplied to the first mixing step and the residual amount of make-up gas may be supplied to all of the steps including the final mixing step, or all (100 mol%) of the make-up gas may be supplied to the first mixing step. When the production method of the present embodiment includes three or more synthesis steps (n≥3), at least part of the make-up gas may be supplied to the first mixing step, and the residual amount of make-up gas may be supplied only to at least part of each step including the final mixing step. In this case, the residual amount of make-up gas may not be supplied to the final mixing step, but only to the other mixing steps.

### (Distribution ratio)

The distribution ratio of the make-up gas is set so that the molar flow rate of the make-up gas contained in the mixed gas (first mixed gas) supplied to the first methanol synthesis step (first synthesis step) is more than 0 mol% and 100 mol% or less, preferably 50 mol% or more and 100 mol% or less, and further preferably 80 mol% or more and 100 mol% or less based on the total volume of the make-up gas. For example, when the distribution ratio of the make-up gas is defined as "(molar volume of make-up gas supplied to the first mixture gas)/(total volume of make-up gas before distribution)," it is more than 0 and 1 or less, preferably 0.5 or more and 1 or less, and further preferably 0.8 or more and 1.0 or less. The distribution ratio of make-up gas mixed with unreacted gas in each mixing step varies in a preferable range depending on the synthesis conditions in each synthesis step and the separation conditions in each separation step, and for example, it can be controlled according to the desired reactor inlet gas composition in the synthesis step and also according to the desired temperature of each reactor. The desired temperature is a temperature in the methanol synthesis reaction described below. This makes it possible to easily control the temperature of the reactor in the synthesis step.

As described above, the final mixing step is supplied with the make-up gas of 0 mol% or more and less than 100 mol% based on the total volume. The amount of the make-up gas supplied to the mixing step after the first mixing step is the residual amount minus the make-up gas supplied to the first mixing step. Specifically, in the case of a two-stage synthesis process, the molar flow rate of the make-up gas supplied to the second mixing step, which corresponds to the final mixing step, is 0 mol% or more and less than 100 mol% based on the total amount of the make-up gas, preferably 0 mol% or more and 50 mol% or less. For example, one aspect can be a production method having two synthesis steps and two condensation and separation steps, using a condensation and separation method as the separation method in the separation step. In this aspect, for example, the outlet gas temperature of the first condensation separation step can be 20°C to 100°C, preferably 40°C to 80°C.

When a third synthesis step or later exists, and the sum of the molar flow rates of the make-up gases supplied to the first and second mixing steps is less than 100% based on the total volume of the make-up gas, the residual make-up gas can be divided appropriately to each methanol mixing step on or after the third mixing step. When the third synthesis steps or later exists, and the sum of the molar flow rates of the make-up gases supplied to the first and second mixing steps is 100% based on the total amount of the make-up gas, it can be configured that the make-up gas is not divided and supplied to each methanol synthesis step on or after the third synthesis step.

### <Flow after introduction into synthesis loop>

In the present embodiment, after the make-up gas is introduced into the synthesis loop, the gas that has passed through at least one synthesis step and at least one separation step passes through the final synthesis step and the final separation step, and the unreacted gas separated in the final separation step is mixed with the make-up gas in the first mixing step and used as synthesis gas in the first synthesis step. The unreacted gas from each separation stage is led to the next mixing step, synthesis step, and separation step, forming a synthesis loop in which each unreacted gas can be introduced into all reactors in series. In order to circulate the gases in this synthesis loop, the production method may include a pressurizing step using a circulator for pressurizing and a depressurizing step, as described above. The production method of the present embodiment includes at least the first mixing step, the first synthesis step and the first separation step, and the final mixing step, the final synthesis step, and the final separation step, as follows.

### <First mixing step>

The "first mixing step" in the present embodiment is a step of mixing the make-up gas distributed in the distribution ratio controlling step with the residual gas after removing a part of gas from the final unreacted gas to obtain the first mixed gas. In the production method of the present embodiment, the make-up gas is introduced into the synthesis loop so that the make-up gas is supplied to each mixing step according to the distribution ratio.

The " residual gas after removing a part of gas from the final unreacted gas" is a part of the final unreacted gas and includes at least hydrogen, carbon monoxide, and carbon dioxide. The M value (inlet M value of the first reaction step) of the make-up gas with which the above-mentioned residual gas is mixed is not particularly limited, but for example, is preferably 1.5 or more and 3 or less, further preferably 1.8 or more and 2.5 or less. The production method of the present embodiment can easily control the reactor inlet M value while maintaining energy efficiency by including the distribution ratio controlling step.

### <First synthesis step>

The "first synthesis step" in the present embodiment is a step for synthesizing methanol from the first mixed gas. In the first synthesis step, the first mixed gas is supplied to the reactor, and a first reaction mixture containing methanol and unreacted gas is generated by the methanol synthesis reaction in the reactor. The first reaction mixture is discharged to the next step, the first separation step. The first mixed gas can be preheated before being supplied to the first synthesis step in the preheating step (first preheating step). In this case, the first reaction mixture obtained by the first synthesis step can be used as a heat source to preheat the first mixed gas in the first preheating step, and the first mixed gas can be preheated by heat exchange with the first reaction mixture. The details of the synthesis step are described below.

### <First separation step>

The "first separation step" in the present embodiment is a step to separate the first unreacted gas from the first reaction mixture obtained in the first synthesis step. In the first separation step, the first reaction mixture is supplied to a separator such as a gas-liquid separator, and the first unreacted gas is separated from the first reaction mixture by known separation means such as condensation. The first unreacted gas separated in the first separation step is discharged to the next mixing step. Details of the separation step are described below.

### <Final Mixing Step>

The "final mixing step" in the present embodiment is a step in which the unreacted gas and the make-up gas distributed in the distribution ratio controlling step are finally mixed to obtain the final mixed gas. Here, " the unreacted gas and the make-up gas distributed in the distribution ratio controlling step are finally mixed " means mixing the make-up gas and the unreacted gas separated from the reaction mixture in the separation step at least one step before the final mixing step. In other words, in the final mixing step, the unreacted gas separated from the reaction mixture in the (n-1)th separation step (the first separation step in the case of a two-stage synthesis process) and make-up gas are mixed, depending on the configuration of the multi-stage synthesis process (n-stage configuration). However, when the distribution ratio of the make-up gas to the final mixing step is 0 mol% in the distribution ratio controlling step, the unreacted gas obtained from the separation step before the final mixing step is supplied directly to the final synthesis step.

### <Final Synthesis step>

The "final synthesis step" in the present embodiment is a step to synthesize methanol from the final mixed gas. The "final synthesis step" in the present embodiment is not particularly limited as long as it is a step of synthesizing methanol from the final mixture gas obtained by mixing the unreacted gas separated from the reaction mixture that has passed through each synthesis step and the make-up gas after the (n-1)th synthesis step (first synthesis step in case of two stage synthesis process), depending on the configuration of the multi-stage synthesis process (n-stage configuration). In the final synthesis step, the final mixed gas is supplied to the reactor, and the methanol synthesis reaction in the reactor produces the final reaction mixture containing methanol and unreacted gas. The final reaction mixture is discharged to the next step, the final separation step.

The final synthesis step is, for example, the step of synthesizing methanol from the second mixed gas (second synthesis step) when the production method of the present embodiment is a two-stage synthesis process. In the case of the three-stage synthesis process, the final synthesis step can be the step of synthesizing methanol from the third mixture gas obtained by mixing the second unreacted gas and the make-up gas (third synthesis step) after separating the second unreacted gas from the second reaction mixture obtained in the second synthesis step. The final mixed gas can be preheated in the preheating step (final preheating step) before being supplied to the final synthesis step. In this case, the final reaction mixture obtained in the final synthesis step can be used as a heat source to preheat the final mixed gas in the final preheating step, and the final mixed gas can be preheated by heat exchange with the final reaction mixture. The details of the synthesis step are described below.

### <Final Separation Step>

The "final separation step" in the present embodiment is a step to separate the final unreacted gas from the final reaction mixture obtained in the final synthesis step. In the final separation step, the final reaction mixture is supplied to a separator such as a gas-liquid separator, and the final unreacted gas is separated from the final reaction mixture by known separation means such as condensation. The final unreacted gas separated in the final separation step is, for example, partially separated and then supplied to the first mixing step to be mixed with the make-up gas.

The final separation step is, for example, a step for separating the second unreacted gas from the second reaction mixture obtained in the second synthesis step (second separation step) if the production method of the present embodiment is a two-stage synthesis process. In the case of the three-stage synthesis process, the step for separating the third unreacted gas from the third reaction mixture obtained in the third synthesis step (the third separation step) can be the final separation step. Details of the separation step are described below.

### <Synthesis step>

As described above, the production method includes at least the first synthesis step and the final synthesis step. The synthesis step is described below.

In each synthesis step, methanol is synthesized from synthesis gas. The reactor used in the synthesis step preferably include, in addition to a catalyst layer, a mechanism (heat-removing mechanism) for removing the heat produced by the reaction from the catalyst layer.

The catalyst used in the synthesis is preferably a methanol synthesis catalyst including copper atoms and zinc atoms as the essential components. Such a catalyst is reduced from the state of oxide by a reducing gas such as hydrogen or carbon monoxide, or a mixed gas including hydrogen and carbon monoxide, and consequently the copper is activated to give catalytic activity to the catalyst. The catalyst may also include, in addition to the copper atoms and the zinc atoms, aluminum atoms and/or chromium atoms as the main third component. The catalyst including copper and zinc as the essential components can be prepared by heretofore known methods. Such a catalyst can be prepared by the methods disclosed in, for example, Japanese Utility Model Publication No. 51-44715, Japanese Patent No. 2695663, Japanese Utility Model Publication No. 06-35401, Japanese Patent Application Laid-Open No. 10-272361, and Japanese Patent Application Laid-Open No. 2001-205089.

A preferable catalyst is a methanol synthesis catalyst including the copper atoms and the zinc atoms in an atomic ratio (copper/zinc) of 2.0 to 3.0, and additionally aluminum atoms. Examples of such a catalyst include the catalysts prepared by the method disclosed in Japanese Patent Application Laid-Open No. 08-299796, and the catalyst disclosed in International Publication No. WO 2011/048976.

Examples of a preferable catalyst include the catalysts used in Examples and Comparative Examples such as Example 2 and Example 3 in International Publication No. 2011/048976. The more preferable atomic ratio (copper/zinc) of the copper atoms and the zinc atoms in the catalyst falls within a range from 2.0 to 3.0. The methanol synthesis catalyst additionally including alumina in a content of 3 to 20% by mass is furthermore preferable. As described above, such a catalyst can be prepared by, for example, the method disclosed in International Publication No. 2011/048976. More specifically, such a catalyst is prepared by, for example, a production method including: a step of producing a precipitate including copper and zinc by mixing an aqueous solution containing copper and an aqueous solution containing zinc and an alkaline aqueous solution; a step of obtaining a mixture by mixing the obtained precipitate and an alumina hydrate having a pseudo boehmite structure; and a step of molding the obtained mixture so as to have a density of 2.0 to 3.0 g/mL. Examples of the molding method include tableting, extrusion molding and tumbling granulation. Furthermore, as the catalyst used in the synthesis step, a catalyst including copper and zinc atoms in an atomic ratio (copper/zinc) of 2.0 to 3.0, and containing 3 to 20% by mass of alumina can be used. Such catalyst can be prepared by a production method inckuding: a step of producing a precipitate including copper and zinc by mixing an aqueous solution containing copper, an aqueous solution containing zinc and an alkaline solution; a step of obtaining a mixture by mixing the precipitate and an alumina hydrate having a pseudo boehmite structure; and a step of molding the obtained mixture so as to have a density of 2.0 to 3.0 g/mL. However, the catalyst used in the present embodiment is not limited to the above-described catalyst and the catalyst prepared by the above-described production method, and may also be other catalysts having the equivalent methanol synthesis activity.

The method for removing heat from the catalyst layer is preferably a method for indirectly exchanging heat between the catalyst layer and pressurized boiling water by using pressurized boiling water as a coolant. Here, the pressurized boiling water means the water boiling so as to utilize the latent heat in the removal of heat from the catalyst layer. Examples of the heat-removing mechanism related to such a heat-removing method include: a cooling mechanism allowing pressurized boiling water to flow in a counter-flow direction or a cocurrent flow direction in relation to the gas flow direction in the catalyst layer; and a cooling mechanism allowing pressurized boiling water to flow in a direction perpendicular to the gas flow direction in the catalyst layer. More specifically, examples of the above-described heat-removing mechanism include: a multitubular reactor having inner tubes parallel to the gas flow direction of the catalyst layer, forming a catalyst layer on the inside of the inner tubes and allowing a coolant to flow on the outside of the inner tubes; the multitubular reactor having the inner tubes parallel to the gas flow direction of the catalyst layer, forming the catalyst layer on the outside of the inner tubes, and allowing the coolant to flow on the inside of the inner tubes; and an interlayer cooling reactor allowing the coolant to flow in the inner tubes disposed so as to be perpendicular to the gas flow direction of the catalyst layer. The temperature of the pressurized boiling water serving as the coolant is preferably 210 to 260□C. The use of the steam produced from the pressurized boiling water is preferably the use as the raw material steam for the steam-reforming reaction of the natural gas.

The control of the reaction temperature of the catalyst layer by the indirect heat exchange with the pressurized boiling water may be performed at least in the final synthesis step (the final reactor); however, it is preferable to control the reaction temperature of the catalyst layer by the indirect heat exchange with the pressurized boiling water in all the synthesis steps. When the pressurized boiling water is used as the coolant in a plurality of reactors, the temperatures of the pressurized boiling water in the respective reactors may be the same as each other or may be different from each other.

The synthesis gas supplied to each synthesis step is preferably preheated to 180 to 260°C with a preheater before being supplied to the synthesis step. The synthesis gas temperature when supplied to each synthesis step is appropriately set according to the type and amount of the catalyst, the type of the reactor, and the reaction pressure, etc. The preferred synthesis gas temperature is 200 to 250°C. From the viewpoint of energy recovery in the reactor, a more preferred gas temperature at the reactor inlet is higher than the temperature of pressurized boiling water used to cool the catalyst layer.

The methanol synthesis reaction in the synthesis step is, as is well known, preferably performed under the conditions that the pressure is 4.9 to 14.7 MPa-G (50 to 150 kg/cm²-G) and the temperature is 200 to 300°C. The pressure and the temperature in the methanol synthesis reaction are more preferably 5.4 to 10.8 MPa-G (55 to 110 kg/cm²-G) and 200 to 280°C, respectively, and furthermore preferably 5.9 to 10.8 MPa-G (60 to 110 kg/cm²-G) and 200 to 270°C, respectively.

When a plurality of reactors has the same catalyst amount, the ratio of the maximum amount to the minimum amount of the methanol production amounts in the respective methanol synthesis steps is preferably 1 to 3 and more preferably 1 to 2.

### <Separation step>

As described above, the production method of the present embodiment includes at least the first separation step and the final separation step. The separation step is described below.

In each separation step, the unreacted gas is separated from the reaction mixture including the reaction product obtained in the synthesis step. In other words, methanol or methanol and water and the unreacted gas included in the reaction mixture are separated. Examples of the separation method include: a condensation separation method in which the outlet gas from the synthesis step is cooled, and the condensed liquid produced by cooling is separated with a gas-liquid separator; and a membrane separation method using a separation membrane, among these the condensation separation method is preferable. In the present embodiment, at least two separation steps (condensation separation steps) using the condensation separation method are provided within the synthesis loop, and one of these steps is preferably the final condensation separation step subsequent to the final synthesis step. The fluid cooled in the condensation separation step is the outlet gas (gaseous reaction mixture) from the synthesis step preceding the condensation separation step, and the outlet gas includes the synthesized methanol. Examples of the method for obtaining the liquid including methanol as a condensed liquid include: an air cooling based on the mutual heat exchange with the synthesis gas supplied to the reactor or an air cooling with an air fin cooler; and a cooling with a coolant such as cooling water or brine. According to the initial temperature before cooling and the target temperature after cooling of the fluid (reaction mixture) being an object to be cooled, the methods for obtaining the condensed liquid are used each alone or in combinations of two or more thereof. Alternatively, the method for obtaining condensed liquid can be combined with the method for obtaining condensed liquid using the cooling associated with expanding the gas with a pressure reducer that can be used in the production method. In general, the obtained condensed liquid is separated by using a gas-liquid separator (hereinafter, also simply referred to as the "separator"). In combining these coolers (condensers) and separators, a combination of one of these coolers and one of these separators may be adopted, or alternatively, a combination of two or more of these cooler and two or more of these separators may also be adopted. Examples of the combination of two or more of these cooler and two or more of these separators include the combination disclosed in Japanese Patent Application Laid-Open No. 61-257934. More specifically, examples of the above-described combinations include a method in which when the reaction mixture obtained by passing through the synthesis step is cooled, and the reaction product mainly including methanol is condensed and separated, the condenser is divided into two stages, the heat transfer surface temperature of the first-stage condenser is set at a temperature equal to or lower than the dew point of the reaction mixture and equal to or higher than the melting point of the paraffins included in the reaction mixture, and the heat transfer surface temperature of the second- stage condenser is set at 60°C or lower.

For example, as an embodiment, here is described a case of a production method using a condensation separation method as the separation method in the separation step, and having two synthesis steps and two condensation separation steps. The first condensation separation step is the step of condensing and separating the outlet gas (gaseous reaction mixture) from the first synthesis step, and is arranged subsequently to the first synthesis step. The first condensation separation step extracts methanol from the synthesis loop by separating preferably 35 to 100 mol%, more preferably 35 to 99 mol%, and furthermore preferably 75 to 96 mol% of the methanol included in the outlet gas from the first synthesis step.

In the condensation separation step, the reaction mixture is cooled until a predetermined amount of the condensed liquid including methanol or methanol and water is produced by cooling. For example, when the fluid (reaction mixture) having a methanol partial pressure of 0.59 to 0.88 MPa-G (6.0 to 9.0 kg/cm2-G) is cooled and condensed, the fluid is cooled preferably at 20 to 100°C and more preferably at 40 to 80°C. In this case, from the viewpoint of improving the methanol yield, in the first condensation separation step, the separation proportion of the methanol included in the outlet gas from the first synthesis step is preferably set at higher than 70 mol%. Moreover, for the reaction control in the subsequent second synthesis step, in the first condensation separation step, the separation proportion of the methanol included in the outlet gas from the first synthesis step is more preferably set at lower than 96 mol%. From the viewpoint of saving the cooling water, the cooling in the first condensation separation step preferably uses only the cooling (air cooling) with an air fin cooler. In this case, the target temperature of the reaction mixture after cooling is preferably 55 to 90°C from the same viewpoint as described above.

As described above, by providing separation steps between a plurality of synthesis steps, the amounts of water supplied to the reactors are reduced in the synthesis steps subsequent to the separation steps. Consequently, as compared with the case where no separation steps are involved, the sintering of the copper particles considered to be the active sites of the catalyst is suppressed, and hence the effect of extending the catalyst service life is assumed. By providing separation steps between a plurality of synthesis steps, and by using the unreacted gas separated in a separation step as the raw material for the synthesis step subsequent to the separation step, the balance between the reaction amounts in the synthesis steps respectively preceding to and subsequent to the separation step concerned is made satisfactory, and consequently the catalysts can be used more effectively. Moreover, in the separation steps between the plurality of synthesis steps, by supplying to the synthesis step subsequent to one of the separation steps the outlet gas from the synthesis step preceding to the separation step concerned without separating 4 to 30 mol% of the methanol included in the outlet gas, the reaction in the subsequent synthesis step can be controlled and the overheating of the catalyst layer can also be suppressed. In this case, the amount of the condensable gas which is not removed in the separation step is increased.

The outlet gas from the final synthesis step is supplied to the final separation step. The final separation step separates at least part of the methanol included in the outlet gas (gaseous reaction mixture) from the final synthesis step. When the condensation separation is adopted in the final separation step, the outlet gas from the final synthesis step is cooled preferably to 20°C to 50°C, for example to 45°C, and is separated into the gas phase (the unreacted gas) and the liquid phase with a gas-liquid separator. The reaction products including methanol separated in the respective separation steps in the synthesis loop are taken out as crude methanol.

### <Purge gas>

Since inert components accumulate in the synthesis loop, it is preferable to remove a part of the final unreacted gas from the system as a purge gas. The purge gas can be taken out at any appropriate position in the synthesis loop.

The outlet of the purge gas in the synthesis loop is positioned preferably at the point of lower pressure in the synthesis loop from the viewpoint of reducing the amount of the gas throughput at the circulator. In addition, from the viewpoint of the carbon yield, a part of the unreacted gas obtained by separating the reaction product from the reaction mixture and discharging the reaction product to the outside of the synthesis loop is preferably divided as a purge gas. In the present embodiment, a part of the unreacted gas can be supplied to the hydrogen recovery unit. Moreover, in the separation steps between the plurality of synthesis steps, by supplying to the synthesis step subsequent to one of the separation steps the outlet gas from the synthesis step preceding to the separation step concerned without separating 4 to 30 mol% of the methanol included in the outlet gas, the reaction in the subsequent synthesis step can be controlled and the overheating of the catalyst layer can also be suppressed. In this case, it is not appropriate to place the circulator at the stage after the separator used in the separation step and further ahead of the preheater prior to the reactor used in the subsequent synthesis step, because condensation may occur in the circulator.

### <Circulation Ratio>

In the present embodiment, the "circulation ratio" is defined as "the molar flow rate of the final unreacted gas (hereinafter also referred to as "circulation gas") mixed with the make-up gas" based on the "molar flow rate of the raw material gas." In the present embodiment, the molar flow rate of the circulation gas is the molar flow rate of the residual gas after removing a part of gas such as purge gas from the final unreacted gas. The reaction product containing methanol separated at each separation step in the synthesis loop is taken out as crude methanol.

The circulation ratio in the methanol synthesis process is defined as the ratio of the molar flow rate of the circulation gas to the molar flow rate of the raw material gas, as described above. In the present embodiment, the circulation ratio is preferably 0.6 to 2.0, and more preferably 0.8 to 1.5. In comparison of the gas composition of the make-up gas and the gas composition of the circulation gas, the make-up gas is higher in the content proportions of carbon monoxide and carbon dioxide, the raw materials for the methanol synthesis, an exothermic reaction, and hence more tends to generate heat in the catalyst layer. Accordingly, by setting the circulation ratio at 0.6 or more, the overheating of the catalyst mainly due to the make-up gas can be further suppressed through the dilution due to the circulation gas. On the other hand, by setting the circulation ratio at 2.0 or less, the energy efficiency in the whole process is improved. This is because the relative increase of the molar flow rate of the make-up gas allows the molar flow rate of hydrogen or the like, intrinsically needing no cooling, to be reduced, and correspondingly, the load on the cooler can be reduced.

### <Examples of the production method and apparatus of the present embodiment>

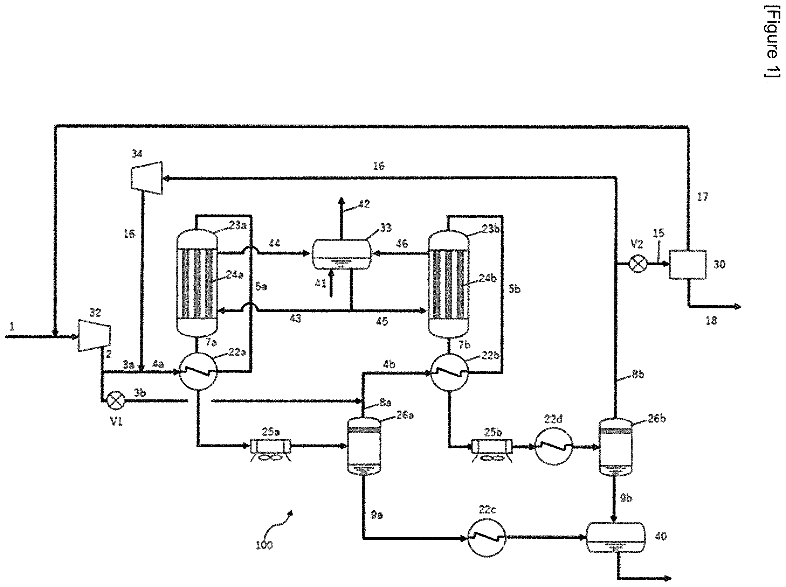

Examples of the production method and apparatus of the present embodiment are described below. Figure 1 is a schematic diagram showing an example of an apparatus used in the methanol production method of the present embodiment. The methanol production apparatus 100 is an apparatus with a two-stage synthesis process. However, the present invention is not limited to the following configuration. The methanol production apparatus 100 includes a first reactor 23a and a second reactor (a final reactor) 23b that synthesize methanol from synthesis gas containing hydrogen, carbon monoxide, and carbon dioxide, a first gas-liquid separator 26a and a second gas-liquid separator (a final gas-liquid separator) 26b that separates unreacted gas from the reaction mixture obtained in the first reactor 23a and the second reactor 23b. The methanol production apparatus 100 includes a synthesis loop with two reactors (23a, 23b) and two gas-liquid separators (separators) (26a, 26b).

The methanol production apparatus 100 is equipped with a hydrogen recovery unit 30. In the synthesis loop, a part of the second unreacted gas (final unreacted gas) separated from the second reaction mixture (final reaction mixture) in the second gas-liquid separator 26b located after the second reactor 23b is supplied to the hydrogen recovery unit 30 via a valve V2 and hydrogen rich gas is recovered. The resulting hydrogen rich gas is supplied into a line 1 via a line 17 and mixed with the raw material gas in the raw material gas mixing unit (confluence of the line 1 and line 17) to become make-up gas. The raw material gas is a low M value gas (M value less than 1), such as a fossil fuel-derived gas or an environmental circular gas.

The methanol production apparatus 100 is equipped with a distribution ratio controller (valve V1). The valve V1 is provided in a line 3b, and by opening and closing the valve V1, the distribution ratio of the make-up gas supplied from a line 2 to a line 3a and the line 3b can be controlled. Specifically, a part of the make-up gas (more than 0 mol% and 100 mol% or less) discharged from a synthetic compressor 32 to the line 2 is supplied to the line 3a and the residual (0 mol% or more and less than 100 mol%) to the line 3b, according to the distribution ratio controlled by the distribution ratio controller (valve V1). Although the distribution ratio controller in the present embodiment is exemplified by the valve V1 on the line 3b, it is not limited to. For example, two valves may be installed on both lines 3a and 3b to control the distribution ratio, or a flow controller may be installed at the junction between the line 2 and the line 3a to control the distribution ratio.

The methanol production apparatus 100 also includes a first mixing unit (at the confluence of the line 3a and a line 16) for mixing the make-up gas distributed by the distribution ratio controller (valve V1) and supplied to the line 3a with the residual gas after removing a part of the final unreacted gas to obtain the first mixed gas, a first reactor 23a for synthesizing methanol from the first mixed gas supplied via lines 4a and 5a, and a first gas-liquid separator 26a for separating the first unreacted gas from the first reaction mixture obtained in the first reactor 23a.

Furthermore, the methanol production apparatus 100 has a second mixing unit (at the confluence of the line 3b and a line 8a) for mixing the first unreacted gas with the make-up gas distributed by the distribution ratio controller and supplied to the line 3b to obtain a second mixed gas, a second reactor 23b for synthesizing methanol from the second mixed gas supplied via lines 4b and 5b, and a second gas-liquid separator 26b for separating the second unreacted gas from the second reaction mixture obtained in the second reactor 23b.

The distribution ratio controller (valve V1) is connected to a control means, which is omitted from the figure, and configured so that the flow rate of the make-up gas supplied to lines 3a and 3b can be controlled by opening and closing the valve V1. The methanol production apparatus 100 can control the M values of the first and second mixed gases supplied to the first and second reactors 23a and 23b by controlling the flow rates of the make-up gas supplied to the lines 3a and 3b by the distribution ratio controller. The controlling of the flow rate of the make-up gas by changing the distribution ratio is not limited, but for example, the M value of each mixed gas at the inlet of each reactor can be monitored, and when the M value changes, the distribution ratio can be changed to control the flow rate of the make-up gas supplied toward each reactor. By controlling the distribution ratio of the make-up gas in this way, the M value of the synthesis gas supplied to each reactor can be controlled within a desired range while suppressing the effect on the energy efficiency of the circulator, etc., compared to, for example, changing the circulation ratio. In the present embodiment, the second reactor 23b, second gas-liquid separator 26b, second reaction mixture, and second unreacted gas correspond to the final reactor, final separator, final reaction mixture, and final unreacted gas, respectively.

The raw material gas, which includes CO, CO₂, and H₂ and has the M value of 1 or less, using environmental circular gas or the like, is mixed with the hydrogen rich gas supplied from the line 17 at the confluence of lines 1 and 17 to form make-up gas. The make-up gas is pressurized to a predetermined pressure by synthetic compressor 32. The pressurized make-up gas is distributed via the line 2 to lines 3a and 3b according to the distribution ratio controlled by the distribution ratio controller (valve 1), flows through each line, and is supplied to the synthesis loop. The make-up gas is mixed at the confluence of the line 3a and the line 16 with the final unreacted gas (circulation gas), a part of which is removed at the valve V2, and supplied to the line 4a. The first mixed gas in the line 4a is supplied to preheater 22a. The first mixed gas, which is the reactor raw material gas, is preheated to a predetermined temperature in the preheater 22a by heat exchange with the reactor outlet gas (first reaction mixture) containing reaction products flowing in a line 7a at the outlet of the reactor 23a, and is supplied from the line 5a to the reactor 23a. The residual of the make-up gas flows in the line 3b.

The reactor 23a has an inner tube 24a, and a methanol synthesis catalyst containing copper and zinc as essential components is filled in the inner tube 24a to form a catalyst layer. Methanol is synthesized in the step that the first mixed gas supplied from the line 5a into the reactor 23a flows through the catalyst layer in the inner tube 24a. The pressure and temperature of the fluid in the catalyst layer may be within the range of reactions and temperatures in the methanol synthesis reaction described above. In the present embodiment, the reactor 23a is described as having an inner tube, but it is not limited to this. The reactor may be configured so that a catalyst is directly filled in the reactor.

The reactor outlet gas (first reaction mixture) containing methanol flowing out of the reactor 23a into the line 7a is cooled in the preheater 22a and then further cooled below the dew point of methanol by a condenser 25a to promote condensation of methanol. The condensed portion of fluid containing condensed methanol is extracted from a line 9a as crude methanol in the gas-liquid separator 26a, and the residual gas phase flows through the line 8a. The crude methanol extracted from the line 9a is further cooled in a heat exchanger 22c, stored in a methanol tank 40, and then discharged outside the system.

The make-up gas flowed through the line 3b is mixed with the first unreacted gas flowed through the line 8a from the gas-liquid separator 26a, and supplied to a preheater 22b from the line 4b as the second mixed gas, which is the reactor raw material gas. The second mixed gas preheated to a predetermined temperature is supplied from the line 5b to the reactor 23b. In this case, the second mixed gas may be configured to be supplied to the reactor 23b after being pressurized to a predetermined reaction pressure by a circulator, which is not shown in the figure.

The reactor 23b has an inner tube 24b, and a methanol synthesis catalyst containing copper and zinc as essential components is filled in the inner tube 24b to form a catalyst layer. Methanol is synthesized in the step that the second mixed gas supplied from the line 5b into the reactor 23b flows through the catalyst layer in the inner tube 24b. The pressure and temperature of the fluid in the catalyst layer may be within the range of reactions and temperatures in the methanol synthesis reaction described above. In the present embodiment, the reactor 23b is described as having an inner tube, but it is not limited to this. The reactor may be configured so that a catalyst is directly filled in the reactor.

The reactor outlet gas (second reaction mixture) containing methanol flowing out of the reactor 23b into the line 7b is cooled in the preheater 22b and then further cooled to a predetermined temperature by a condenser 25b, whereby the methanol is further condensed. The second reaction mixture containing condensed methanol is further cooled in a heat exchanger 22d, and then the condensed portion is extracted from a line 9b as crude methanol in the gas-liquid separator 26b, while the gas phase portion (second unreacted gas) flows through the line 8b. However, the present embodiment is not limited to such an aspect, and it may be an aspect to cool methanol in the condenser 25b only. Crude methanol extracted from the line 9a is stored in the methanol tank 40 and then discharged outside the system.

Meanwhile, a part of the second unreacted gas in an amount that results in a predetermined circulation ratio flows through the line 16 as circulation gas, merges with the make-up gas flowing through the line 3a, and is circulated to the reactor 23a. The residual unreacted gas is supplied from the synthesis loop through a line 15 to the hydrogen recovery unit 30. In the hydrogen recovery unit 30, hydrogen is recovered from a part of the unreacted gas and discharged into the line 17 as hydrogen rich gas, and the second unreacted gas after the hydrogen was recovered is discharged out of the system via a line 18 as purge gas to remove inert components that accumulate in the synthesis loop. As described above, the hydrogen rich gas flowing through the line 17 is mixed with the raw material gas flowing through the line 1. On the other hand, the circulation gas flowing through the line 16 is mixed with the make-up gas flowing through the line 3a after being pressurized in a circulator 34, and is supplied to the preheater 22a from the line 4a as the first mixed gas, which is the reactor raw material gas. The first mixed gas preheated to a predetermined temperature is supplied from the line 5a to the reactor 23a.

In the present embodiment, the apparatus can be configured so that a steam drum 33 is provided and the reaction temperature of the catalyst layer is controlled by indirect heat exchange with pressurized boiling water in the reactor 23a (first reactor), and the pressurized boiling water is at least partially circulated between the reactor 23b (second reactor; final reactor) and the reactor 23a (first reactor) and the steam drum. As shown in Figure 1, cooling of the catalyst layers in the reactors 23a and 23b is accomplished by introducing boiler water from the steam drum 33 into the reactors 23a and 23b, respectively, from lines 43 and 45, which are used as pressurized boiling water, and the resulting steam -containing fluid is recovered from lines 44 and 46, respectively. The steam produced by the heat of reaction is taken out of the steam drum 33 to a line 42, and water is supplied from a line 41 to the steam drum 33 to compensate for the amount of steam. The steam extracted from the line 42 can also be used as feedstock steam for the steam reforming reaction when the raw material gas is generated from natural gas.

### (Other aspects of the present embodiment)

Although an example of the present embodiment has been described above using the figure, the present invention is not limited to the above-mentioned aspects. For example, although Figure 1 describes a methanol synthesis apparatus with a two-stage synthesis process, the methanol synthesis apparatus of the present embodiment can be a methanol synthesis apparatus with a three-stage or more synthesis process. In the three-stage or more synthesis process, the methanol synthesis apparatus is configured to supply make-up gas to the first to nth reactors (final reactors) in accordance with the distribution ratio to each mixing step by a distribution ratio controller, and a hydrogen recovery unit separating hydrogen rich gas from a part of the final unreacted gas is installed at the rear stage of the nth gas-liquid separator (final separator). In this case, the distribution ratio controller is configured to be able to supply the make-up gas of more than 0 mol% and 100 mol% or less to the first reactor and to supply the residual make-up gas to the respective mixed gases other than the first mixed gas. Thus, even in the case of the three-stage or more synthesis process, by using a low M value gas as the raw material gas, using a mixture of the gas and hydrogen rich gas as the make-up gas, and controlling the distribution ratio of the make-up gas supplied to each reactor, the M value of the synthesis gas supplied to each reactor can be controlled within a desired range while suppressing the effect on the energy efficiency of the circulator etc., in comparison with changing the circulation ratio.

### Examples

Hereinafter, the present invention is described in more detail by way of Examples and Comparative Examples of the methanol synthesis plant design, but the present invention is not limited to these Examples and Comparative Examples.

### <Common Conditions>

In each of the examples and comparative examples, a simulation was conducted in which the temperature, pressure, and material flow rate of the fluid flowing through each line were adjusted to the values listed in the respective tables to achieve a methanol production amount of 5050 tons/day. The carbon yield (actual yield/theoretical maximum carbon yield) was set to 96%. Note that when the M value is 1 or less, the theoretical maximum carbon yield is less than 100% (specifically, when the M value is 1 or less, the M value itself becomes the theoretical maximum carbon yield) because the amount of hydrogen is less than stoichiometric amount.

In this case, the catalyst used for methanol synthesis was the catalyst prepared by the method disclosed in Example 3 of International Publication No. WO 2011/048976 (methanol synthesis catalyst A), with a catalyst volume of 202.5 tons.

The effects in the results for each example and comparison example are as follows.
- Synthesis pressure: Synthesis pressure required to meet the production amount of 5050 t/day.
- Circulation ratio: circulation gas flow rate / raw material gas flow rate
- Distribution ratio: molar volume of make-up gas supplied to the first mixed gas / total volume of make-up gas before distribution
- Energy index: The total shaft power (kW) of the reference example (synthesis compressor power + circulator power) was set as 100%, and the total shaft power of the example or comparison example was used as an index of energy consumption in the methanol synthesis.

### <Composition of raw material gas and M value>

The table below shows the relationship between the flow rate and composition of the raw material gas with each M value used in the examples and comparative examples and the M value.

### [Table 1]

**[Table 1]**

| | | M=0.800 | | M=0.850 | | M=0.699 | | M=0.949 | | M=0.971 | | M=1.025 | | M=1.040 | | M=1.357 | |
|---|---|---|---|---|---|---|---|---|---|---|---|---|---|---|---|---|---|
| | Flow Rate of Raw Material Gas | 24433.1 | kmol/h | 23955.5 | kmol/h | 23534.2 | kmol/h | 23168.1 | kmol/h | 23006.8 | kmol/h | 23498.1 | kmol/h | 23980.5 | kmol/h | 29608.3 | kmol/h |
| | Component | Mol frac | kmol/h | Mol frac | kmol/h | Mol frac | kmol/h | Mol frac | kmol/h | Mol frac | kmol/h | Mol frac | kmol/h | Mol frac | kmol/h | Mol frac | kmoUh |
| | METHANE | 0.0397 | 970.0 | 0.0397 | 951.0 | 0.0397 | 934.3 | 0.0397 | 919.8 | 0.0397 | 913.4 | 0.0168 | 393.6 | 0.0397 | 952.0 | 0.0310 | 918.9 |
| | CO | 0.2880 | 7036.7 | 0.2743 | 6571.0 | 0.2626 | 6180.1 | 0.2508 | 5810.5 | 0.2463 | 56666 | 0.2068 | 4859.1 | 0.2079 | 4985.5 | 0.1514 | 4483.7 |
| Composition of Raw Material Gas | CO2 | 0.0618 | 1510.0 | 0.0615 | 1473.3 | 0.0607 | 1428.5 | 0.0605 | 1401.7 | 0.0600 | 1380.4 | 0.0844 | 1982.5 | 0.0774 | 1856.1 | 0.0796 | 2358.0 |
| | H2 | 0.6095 | 14892.0 | 0.6235 | 14936.3 | 0.6360 | 14967.8 | 0.6480 | 15012.9 | 0.6530 | 15023.4 | 0.6836 | 16064.1 | 0.6740 | 16162.8 | 0.7353 | 21772.0 |
| | N2 | 0.0000 | 0.0 | 0.0000 | 0.0 | 0.0000 | 0.0 | 0.0000 | 0.0 | 0.0000 | 0.0 | 0.0051 | 119.5 | 0.0000 | 0.0 | 0.0014 | 40.4 |
| | H2O | 0.0010 | 24.4 | 0.0010 | 24.0 | 0.0010 | 23.5 | 0.0010 | 23.2 | 0.0010 | 23.0 | 0.0034 | 79.3 | 0.0010 | 24.0 | 0.0012 | 35.3 |
| | Total | 1.00000 | 24433.11 | 1.00000 | 23955.51 | 1.00000 | 23534.24 | 1.00000 | 23108.05 | 1.00000 | 23006.79 | 1.00000 | 23498.12 | 1.0000 | 23980.47 | 1.0000 | 29608.34 |
| M Value (M=H2/(2*CO + 3*CO2)) | | 0.800 | | 0.850 | | 0.899 | | 0.949 | | 0.971 | | 1.025 | | 1.040 | | 1.357 | |

### <Comparison of M value of raw material gas and energy consumption>

### [Example 1]

A simulation of methanol synthesis was conducted using the methanol production apparatus 100 in the two-stage synthesis process shown in Figure 1, with a raw material gas with M value = 0.971 and catalyst activity set to a low activity at the end of operation. The results are shown in the table below.

### [Comparative Examples 1, 2, and 3]

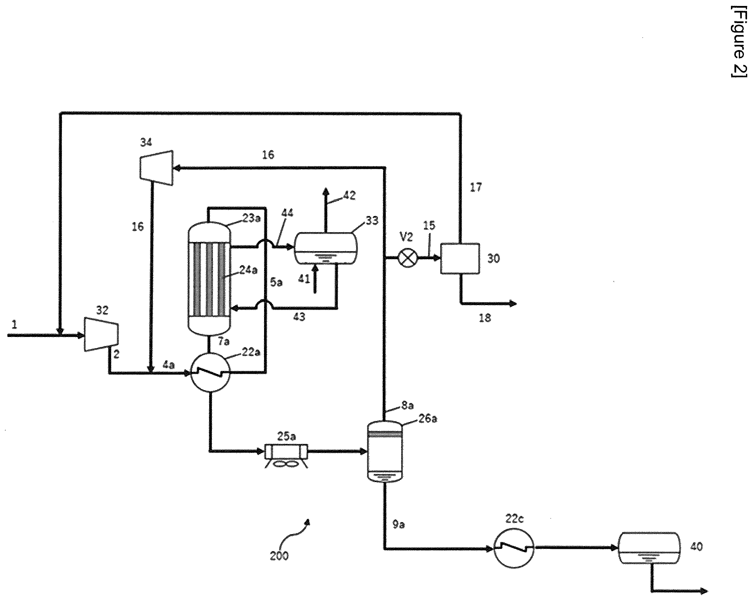

In Comparative Example 1, a simulation of methanol synthesis was conducted in the same way as in Example 1, except that a methanol production apparatus 200 in the single-stage synthesis process shown in Figure 2 was used. In Figure. 2, elements with the same symbols as in the methanol production apparatus 100 in Figure 1 indicate the same elements, etc. as the elements in Figure 1.

In Comparative Example 2, a simulation of methanol synthesis was conducted in the same way as in Example 1, except that a raw material gas with M value = 1.025 was used.

In Comparative Example 3, a simulation of methanol synthesis was conducted in the same way as in Example 1, except that a raw material gas with M value = 1.357 was used.

The results are shown in the table below.

### [Table 2]

**[Table 2]**

| | Example 1 | Com. Exam. 1 | Com. Exam. 2 | Com. Exam. 3 |
|---|---|---|---|---|
| M value of raw material gas | 0.971 | | 1.025 | 1.357 |
| Number of stage(s) of synthesis | Two stages | Single stage | Two stages | Two stages |
| Distribution rato | 1.0 | | | |
| Circulation ratio | 1.4 | | | |
| M value of synthesis gas at inlet of first reactor | 1.85 | 1.96 | 2.60 | 6.89 |
| Maximum temperature of first reactor [C°] | 270.77 | 289.19 | 270.01 | 266.3 |
| Synthesis pressure of synthesis gas at inlet of first reactor [K/G] | 96.16 | 117.70 | 105.50 | 116.3 |
| Power index (vs. Example 1) | 100.0% | 114.1% | 103.6% | 252.1% |

As shown in the table above, Comparative Example 1 using the methanol production method of the single-stage synthesis process, and Comparative Examples 2 and 3 using the raw material gas with high M value (M value > 1), had a higher total shaft power and power index compared to Example 1.

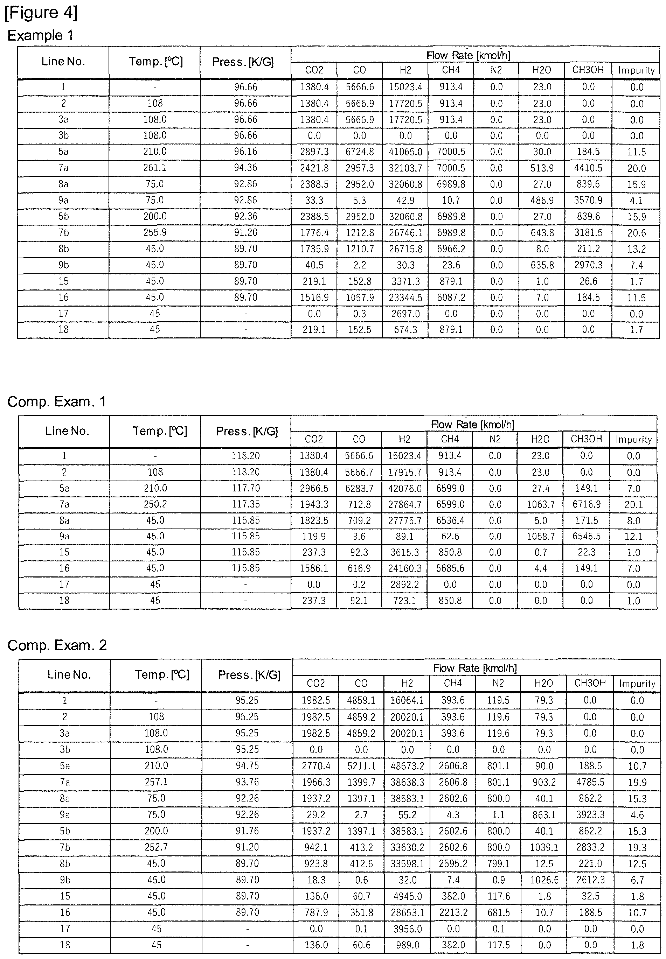

These results indicate that the methanol production method using low M value gas and a two-stage synthesis process has higher energy efficient and can synthesize methanol more efficiently than the production method using low M value gas and a single-stage synthesis process, or the production method using high M value gas and a two-stage synthesis process. Figure 4 shows the results of the material balance in Example 1, Comparative Example 1, and Comparative Example 2. In each table, the line numbers correspond to the line numbers in the figures in the corresponding example and comparative example.

### <Comparison of make-up gas distribution ratio and energy consumption>

### [Example 2]

A simulation of methanol synthesis was conducted using the methanol production apparatus 100 of the two-stage synthesis process shown in Figure 1 and the raw material gas with M value = 0.971, varying the distribution ratio of the make-up gas to the first reactor in the range of 1.0 to 0.5, and setting the catalyst activity to high activity in the initial stage of operation. The results are shown in the table below.

### [Comparative Example 4]

A simulation of methanol synthesis was conducted in the same way as in Example 2, except that the distribution ratio of the make-up gas toward the second reactor was set to 0 (all (100 mol%) of the make-up gas was distributed to the second mixed gas). The results are shown in the table below.

### [Table 3]

**[Table 3]**

| | Example 2 | | | | | | Com. Exam. 4 |
|---|---|---|---|---|---|---|---|
| | Exam. 2-1 | Exam. 2-2 | Exam. 2-3 | Exam. 2-4 | Exam. 2-5 | Exam. 2-6 | |
| M value of raw material gas | 0.971 | | | | | | 0.971 |
| Distribution rato | 1.0 | 0.9 | 0.8 | 0.7 | 0.6 | 0.5 | 0.0 |
| Circulation ratio | 1.4 | | | | | | 1.4 |
| M value of synthesis gas at inlet of first reactor | 1.85 | 1.91 | 1.99 | 2.07 | 2.17 | 2.29 | 3.72 |
| Maximum temperature of first reactor [C°] | 276.28 | 276.61 | 276.96 | 277.29 | 277.61 | 277.80 | 273.95 |
| Synthesis pressure of synthesis gas at inlet of first reactor [K/G] | 73.37 | 74.25 | 75.31 | 76.56 | 78.07 | 79.94 | 94.84 |
| Power index (vs. Example 1) | 100.0% | 100.6% | 101.4% | 102.5% | 103.9% | 105.7% | 120.9% |

As shown in the table above, compared to Comparative Example 4, where all of the make-up gas was distributed to the second mixture gas, the total shaft power was small and the power index was low in each of Example 2-1 through Example 2-6, where the distribution ratio of the make-up gas towards the first reactor was varied in the range of 1.0 to 0.5 (50 to 100 mol% of the make-up gas was supplied to the first reactor).

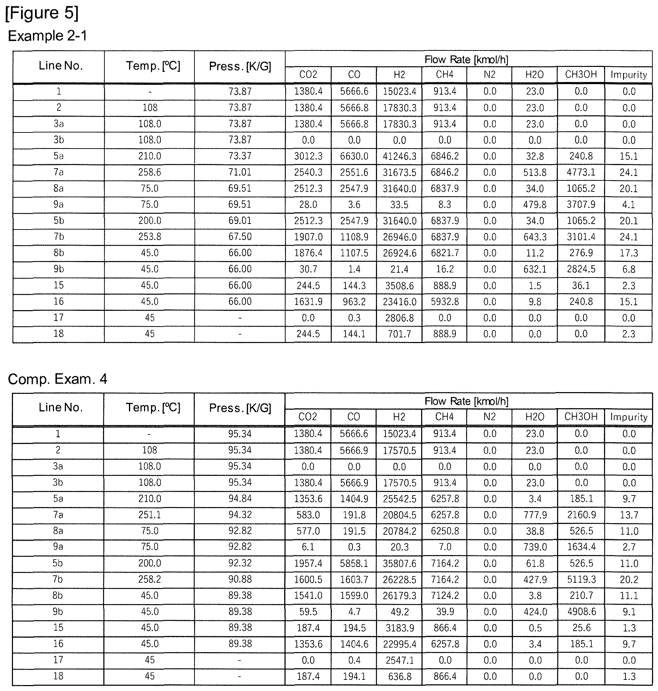

These results indicate that the methanol production method in which the distribution ratio is controlled so that the amount of the make-up gas supplied toward the first mixed gas is more than 0 mol% (in particular, 50 to 100 mol%) has higher energy efficient and can synthesize methanol more efficiently than the production method in which all the make-up gas is distributed to the second mixed gas. Figure 5 shows the results of the material balance corresponding to Example 2-1 and Comparative Example 4. In each table, the line numbers correspond to the line numbers in the figures of the corresponding examples and comparative examples.

### <Relationship between M value of raw material gas and hydrogen recovery unit>

### [Example 3]

A simulation of methanol synthesis was conducted using the methanol production apparatus 100 in the two-stage synthesis process shown in Figure 1, with a raw material gas with M value = 0.971, and catalyst activity set to a high activity at the initial of operation. The results are shown in the table below.

### [Comparative Examples 5, 6, and 7]

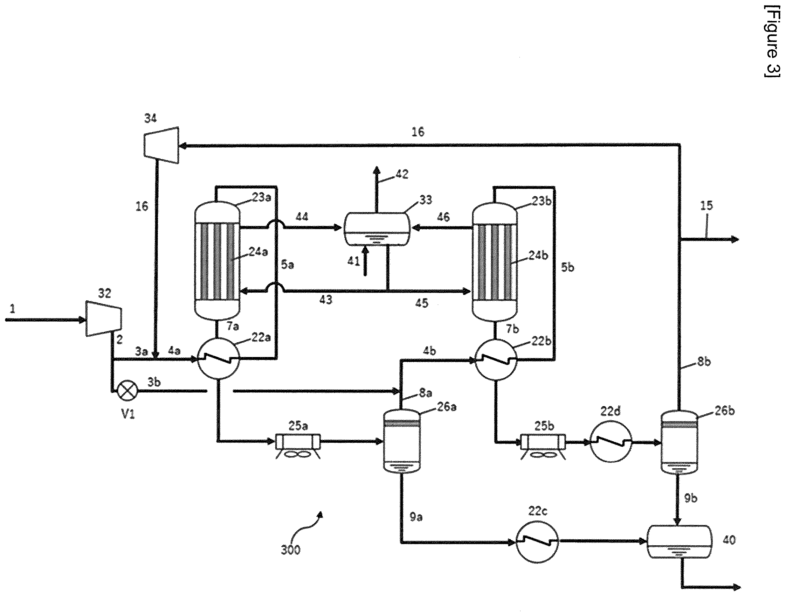

In Comparative Example 5, a simulation of methanol synthesis was conducted in the same way as in Example 3, except that a methanol production apparatus 300 in the two-stage synthesis process (without hydrogen recovery unit) shown in Figure 3 was used. In Figure 3, elements with the same symbols as in the methanol production apparatus 100 in Figure 1 indicate the same elements, etc. as the elements in Figure 1.

In Comparative Example 6, a simulation of methanol synthesis was conducted in the same way as in Example 1, except that a raw material gas with M value = 1.040 was used.

In Comparative Example 7, a simulation of methanol synthesis was conducted in the same way as in Comparative Example 5, except that a raw material gas with M value = 1.040 was used.

The results are shown in the table below.

### [Table 4]

**Table 4]**

| | Example 3 | Com. Exam. 5 \| | Com. Exam. 6 | Com. Exam. 7 |
|---|---|---|---|---|
| Hydrogen recovery unit | Present | Absent | Present | Absent |
| M value of raw material gas | 0.971 | | 1.040 | |
| Distribution rato | 0.8 | | | |
| Circulation ratio | 1.4 | | | |
| M value of synthesis gas at inlet of first reactor | 1.99 | 1.00 | 3.05 | 1.69 |
| Maximum temperature of first reactor [C°] | 276.96 | 282.30 | 280.86 | 283.91 |
| Synthesis pressure of synthesis gas at inlet of first reactor [K/G] | 75.31 | 101.63 | 90.06 | 108.97 |
| Power index (vs. Example 3) | 100.0% | 128.6% | 128.8% | 140.3% |

As shown in the table above, Comparative Examples 5 and 7 not using a hydrogen recovery unit, and Comparative Example 6 using the raw material gas with high M value (M value > 1), have a higher total shaft power and power index compared to Example 3.

From these results, the methanol production method using the hydrogen recovery unit and low M-value gas has higher energy efficient and can synthesize methanol more efficiently than the production method using low M value gas and not using a hydrogen recovery unit, the production method using high M value gas and a hydrogen recovery unit, and the production method using high M-value gas and not using a hydrogen recovery unit.

Furthermore, taking into consideration the results of comparison between with and without a hydrogen recovery unit when using a raw material gas with M value = 1.040, which is outside the low M value range (comparison of Comparison Examples 6 and 7), the comparison results of Examples 4 and 5 using a raw material gas with low M value show that the range of improvement depending on with or without a hydrogen recovery unit is large. Therefore, it can be said that when a low M value raw material gas is used, the improvement range of the total shaft power due to the installation of the hydrogen recovery unit is large, and the installation of the hydrogen recovery unit is highly effective.

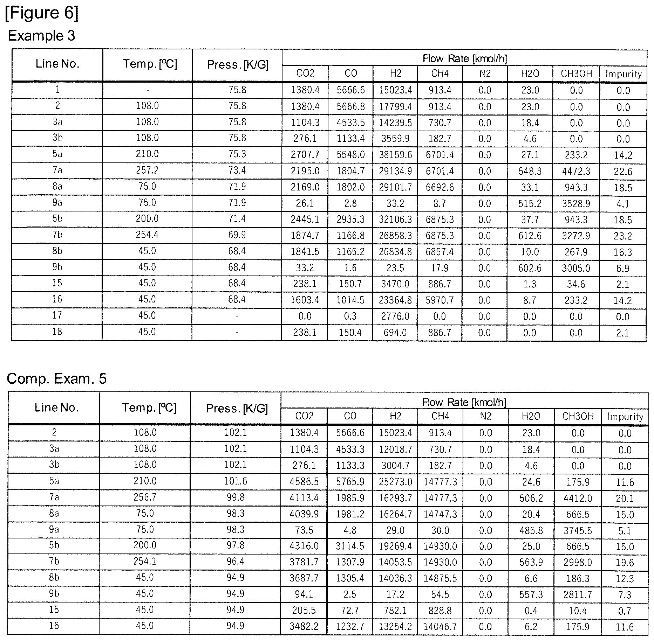

Figure 6 shows the material balance corresponding to Example 3 and Comparison Example 5. In each table, the line numbers correspond to the line numbers in the figure in the corresponding Example and Comparative Example.

### <Relationship between controlling of distribution ratio and circulation ratio and energy consumption>

### [Example 4]

As Example 4, a simulation of methanol synthesis was conducted using the methanol production apparatus 100 of the two-stage synthesis process shown in Figure 1 and the raw material gas with M value = 0.971, varying the distribution ratio of the make-up gas to the first reactor in the range of 1.0 to 0.5, and setting the catalyst activity to high activity in the initial stage of operation, in the same way as in Example 2.

In Example 4-1, a simulation of methanol synthesis was conducted in the same way as in Example 4, except that the distribution ratio was fixed at 1.0 (100% of the make-up gas was distributed to the first reactor) and the circulation ratio was varied in the range of 1.0 to 1.8.

In Example 4-2, a simulation of methanol synthesis was conducted in the same way as in Example 4, except that the distribution ratio was fixed at 0.8 (80% of the make-up gas was distributed to the first reactor) and the circulation ratio was varied in the range of 1.0 to 1.6.

The results are shown in the table below.

### [Table 5]

**[Table 5]**

| | Example 4 (=Example 2) | | | | | | Example 4-1 | | | | | Example 4-2 | | | |
|---|---|---|---|---|---|---|---|---|---|---|---|---|---|---|---|
| | A | B | C | D | E | F | A | B | C | D | E | A | B | C | D |
| M value of raw material gas | 0.971 | | | | | | 0.971 | | | | | 0.971 | | | |
| Distribution rato | 1.4 | | | | | | 1.0 | 1.2 | 1.4 | 1.6 | 1.8 | 1.0 | 1.2 | 1.4 | 1.6 |
| Circulation ratio | 1.0 | 0.9 | 0.8 | 0.7 | 0.6 | 0.5 | 1.0 | | | | | 0.8 | | | |
| M value of synthesis gas at inlet of first reactor | 1.85 | 1.91 | 1.99 | 2.07 | 2.17 | 2.29 | 1.72 | 1.79 | 1.85 | 1.90 | 1.95 | 1.85 | 1.92 | 1.99 | 2.04 |
| Maximum temperature of first reactor [C°] | 276.28 | 276.61 | 276.96 | 277.29 | 277.61 | 277.80 | 283.17 | 279.12 | 276.23 | 274.12 | 272.52 | 284.89 | 280.22 | 276.96 | 274.51 |
| Synthesis pressure of synthesis gas at inlet of first reactor [K/G] | 73.37 | 74.25 | 75.31 | 76.56 | 78.07 | 79.94 | 78.09 | 75.35 | 73.51 | 72.28 | 71.47 | 8102 | 77.71 | 75.31 | 73.89 |
| Power index (vs. Example 4-A) | 100.0% | 100.6% | 101.4% | 102.5% | 103.9% | 105.7% | 100.8% | 99.6% | 100.2% | 102.1% | 105.4% | 103.9% | 101.8% | 101.4% | 105.2% |

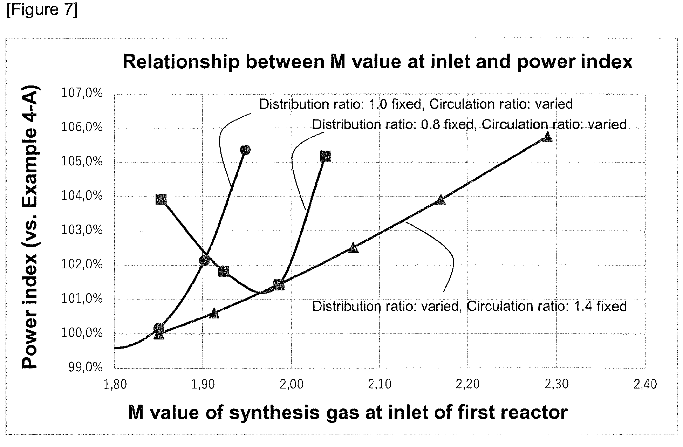

As shown in the table above and Figure 7, a comparison of the power index for the similar M value of the synthesis gas at the inlet of the first reactor shows that the energy consumption is always lower in Example 4 than in Examples 4-1 to 4-2. Therefore, when controlling the M value at the reactor inlet to the higher M value side, from the viewpoint of the energy consumption, it is also advantageous to control the distribution ratio of the make-us gas as in Example 4 more than to change the circulation ratio as in Examples 4-1 and 4-2 because the total shaft power required to control the M value at the reactor inlet to the higher M value is smaller.

In this regard, as described above, as disclosed in, for example, Hiromichi Arai (1978), "Hydrogenation of Carbon Monoxide with Solid Catalysts (I)," Oil Chemistry Vol. 27, No. 8, pp. 491-500), etc., it is known that by-products (ethanol, acetone, methyl ethyl ketone, paraffin, etc.) produced in methanol synthesis increase or decrease depending on CO content and H/C ratio. The results of the examples show that, according to the present invention, by controlling the distribution ratio of the make-up gas, the increase or decrease of by-products can be controlled while controlling the increase or decrease of energy consumption.

### <Relation between M value of raw material gas and energy consumption>

### [Example 5]

A simulation of methanol synthesis was conducted using the methanol production apparatus 100 of the two-stage synthesis process shown in Figure 1 and the raw material gas with M value = 0.800 to 0.971, and setting the catalyst activity to low activity at the end of operation, the distribution ratio to the range of 0.5 to 1.0 and the circulation ratio to the ranges of 1.0 to 2.0. The results are shown in the table below.

### [Table 6]

**[Table 6]**

| | Example 5-1 | Example 5-2 | Example 5-3 | Example 5.4 | Example 5-5 | Example 5-6 | Example 5-7 | Example 5-8 | Example 5-9 | Example 5-10 | Example 5-11 | Example 5-121 | Example 5-131 |
|---|---|---|---|---|---|---|---|---|---|---|---|---|---|
| M value of raw material gas | 0.850 | 0.899 | 0.949 | 0.971 | 0.850 | 0.899 | 0.909 | 0.971 | 0.800 | 0.850 | 0.899 | 0.949 | 0.971 |
| Distribution rata | 0.8 | | | | 0.8 | | | | 0.8 | | | | |
| Circulation ratio | 1.0 | | | | 1.4 | | | | 2.0 | | | | |
| M value of synthesis gas at inlet of first reactor | 129 | 1.47 | 1.71 | 1.86 | 1.27 | 1.48 | 1.79 | 1.99 | 1.05 | 1.23 | 1.48 | 1.86 | 2.13 |
| Maximum temperature of first reactor [C°] | 271.68 | 272.63 | 274.55 | 276.13 | 269.24 | 269.63 | 270.53 | 271.32 | 267.49 | 267.41 | 267.44 | 267.70 | 268.03 |
| Synthesis pressure of synthesis gas at inlet of first reactor [K/G] | 81.61 | 86.52 | 95.34 | 101.91 | 80.44 | 84.68 | 92.53 | 9846 | 78.81 | 80.70 | 84.29 | 91.40 | 97.01 |
| Power index | 101.8% | 100.7% | 106.0% | 115.6% | 101.3% | 100.0% | 105.9% | 109.5% | 115.8% | 107.1% | 110.0% | 111.2% | 112.0% |

| | Example 5-14 | Example 5-15 | Example 5-16 | Example 5-17 | Example 5-18 | Example 5-19 | Example 5.20 | Example 5-21 | Example 5-23 | | | | |
|---|---|---|---|---|---|---|---|---|---|---|---|---|---|
| M value of raw material gas | 0.850 | 0.899 | 0.949 | 0.971 | 0.800 | 0.850 | 0.899 | 0.949 | 0.971 | | | | |
| Distribution rato | 0.5 | | | | 1.0 | | | | | | | | |
| Circulation ratio | 1.4 | | | | 1.4 | | | | | | | | |
| M value of synthesis gas at inlet of first reactor | 1.35 | 1.61 | 2.01 | 2.29 | 1.07 | 1.23 | 1.42 | 1.69 | 1.85 | | | | |
| Maximum temperature of first reactor [C°] | 270.24 | 270.59 | 271.42 | 272.18 | 268.56 | 268.74 | 269.14 | 270.04 | 270.81 | | | | |
| Synthesis pressure of synthesis gas at inlet of first reactor [K/G] | 80.84 | 86.14 | 95.92 | 103.38 | 78.39 | 80.56 | 84.31 | 91.29 | 96.57 | | | | |
| Power index | 102.8 % | 102.5% | 108.2% | 121.6% | 103.8 % | 102.2% | 102.4% | 105.3% | 108.2% | | | | |

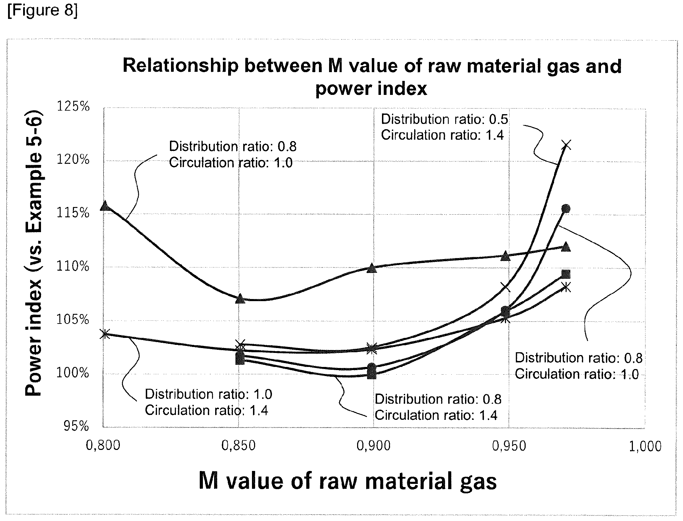

As shown in the table above and Figure 8, a comparison of the examples in which the distribution ratio and circulation ratio were fixed and the M value of the raw material gas was varied shows that in all examples, the total shaft power was minimum with the M value of the raw material gas in the range of 0.850 to 0.950, and the minimum value of the power index exists within the same M value range. Therefore, it can be seen that the most efficient M value composition range for the methanol synthesis production method of the present embodiment exists within the range of 0.850 to 0.950 of the M value of the raw material gas.

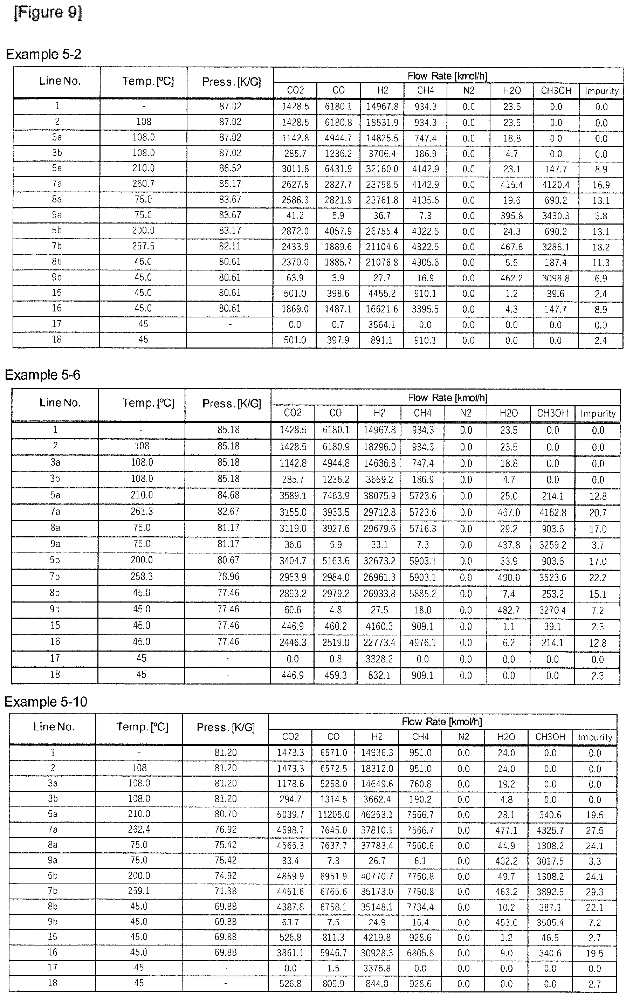

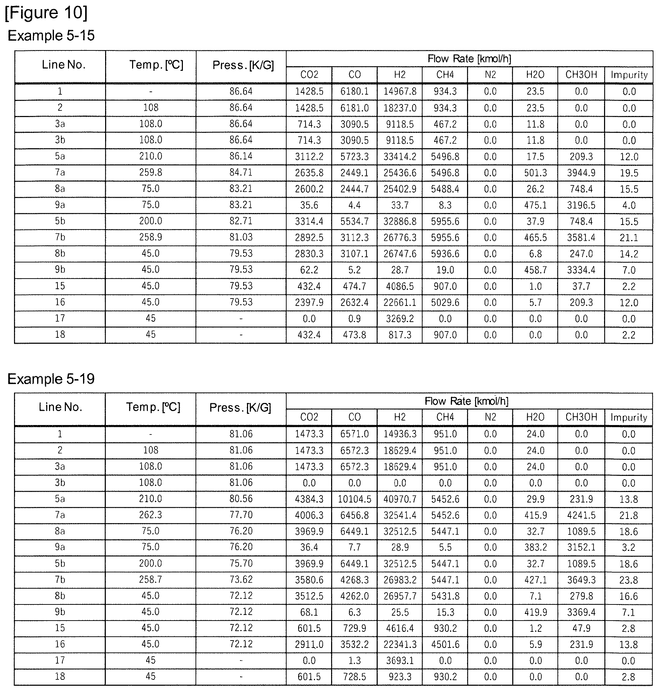

Figures 9 and 10 shows the material balance corresponding to Example 5 (Example 5-2, Example 5-6, Example 5-10, Example 5-15, and Example 5-19). In each table, the line numbers correspond to the line numbers in the figure in the corresponding examples.

### Reference signs list

23a, 23b ... Reactor, 26a, 26b ... Gas-liquid separator, 30 ... Hydrogen recovery unit, V1 ... Valve (distribution ratio controller)

## Claims

1. A method for producing methanol comprising:
a synthesis step of synthesizing methanol from a synthesis gas comprising hydrogen, carbon monoxide and carbon dioxide; and
a separation step of separating an unreacted gas from a reaction mixture obtained by passing through the synthesis steps,
wherein the method comprises:
a synthesis loop comprising at least two of the synthesis steps and at least two of the separation steps,
a raw material gas mixing step of obtaining a make-up gas by mixing a hydrogen rich gas obtained from a hydrogen recovery unit to which at least part of unreacted gas separated from a final reaction mixture in a final separation step is supplied with a raw material gas comprising at least hydrogen and carbon dioxide and having M value (M value=[H₂]/([2CO]+[3CO₂])) being 1 or less in the synthesis loop,
a distribution ratio controlling step of controlling a distribution ratio of the make-up gas, supplying more than 0 mol % and 100 mol % or less of the make-up gas to a first mixing step in accordance with the distribution ratio, and supplying 0 mol % or more and less than 100 mol % of the make-up gas to a final mixing step,
the first mixing step of obtaining a first mixed gas by mixing the make-up gas distributed from the distribution ratio controlling step with a residual gas obtained by removing a part of gas from the final unreacted gas,
a first synthesis step of synthesizing the methanol from the first mixed gas,
a first separation step of separating the first unreacted gas from the first reaction mixture obtained in the first synthesis step,
the final mixing step of obtaining a final mixed gas by finally mixing the unreacted gas with the make-up gas distributed from the distribution ratio controlling step,
the final synthesis step of synthesizing the methanol from the final mixed gas, and the final separation step of separating the final unreacted gas from the final reaction mixture obtained in the final synthesis steps.

2. The method for producing methanol according to claim 1, wherein the M value is 0.8 or more and 0.95 or less.

3. The method for producing methanol according to claim 1 or 2, wherein the raw material gas comprises an environmental circular gas.

4. The method for producing methanol according to any one of claims 1 to 3, wherein 50 mol % or more and 100 mol % or less of the make-up gas is distributed into the first mixing step.

5. The method for producing methanol according to any one of claims 1 to 4, wherein the make-up gas obtained in the raw material gas mixing step is pressurized.

6. The method for producing methanol according to any one of claims 1 to 5, wherein a circulating ratio that is a ratio of a molar flow rate of the make-up gas to a molar flow rate of the final unreacted gas mixed with the make-up gas is 0.6 to 2.0.

7. The method for producing methanol according to claim 6, wherein the circulation ratio is 0.8 to 1.5.

8. An apparatus for producing methanol comprising:
a reactor for synthesizing methanol from a synthesis gas comprising hydrogen, carbon monoxide and carbon dioxide; and
a separator for separating an unreacted gas from a reaction mixture obtained in the reactors,
wherein the apparatus comprises:
a synthesis loop comprising at least two of the reactors and at least two of the separators,
a raw material gas mixing unit for obtaining a make-up gas by mixing a hydrogen rich gas obtained from a hydrogen recovery unit to which at least part of unreacted gas separated from a final reaction mixture in a final separator is supplied with a raw material gas comprising at least hydrogen and carbon dioxide and having M value (M value=[H₂]/([2CO]+[3CO₂])) being 1 or less in the synthesis loop,
a distribution ratio controller for controlling a distribution ratio of the make-up gas, supplying more than 0 mol % and 100 mol % or less of the make-up gas to a first mixing unit in accordance with the distribution ratio, and supplying 0 mol % or more and more than 100 mol % of the make-up gas to a final mixing unit,
the first mixing unit for obtaining a first mixed gas by mixing the make-up gas distributed from the distribution ratio controller with a residual gas obtained by removing a part of gas from the final unreacted gas,
a first reactor for synthesizing the methanol from the first mixed gas,
a first separator for separating the first unreacted gas from the first reaction mixture obtained in the first reactor,
the final mixing unit for obtaining a final mixed gas by finally mixing the unreacted gas with the make-up gas distributed from the distribution ratio controller,
the final reactor for synthesizing the methanol from the final mixed gas, and
the final separator for separating the final unreacted gas from the final reaction mixture obtained in the final reactors.
